# EUROPEAN PATENT SPECIFICATION

(11) **EP 3 921 029 B1**
(45) Date of publication and mention of the grant of the patent: **30.07.2025**
(21) Application number: 20753233.4
(22) Date of filing: 07.02.2020
(51) Int. Cl.: A61P 19/08, A61K 38/19, A61K 38/18, C07K 14/52, C07K 14/51, C07K 16/24, C12N 5/00, A61L 27/36, A61L 27/52, A61L 27/54, C07K 14/475, C07K 14/525, C12N 5/077, C12N 9/14, A61K 31/46, C07K 16/00

(54) **COMPOSITION COMPRISING CCL5 FOR USE IN TREATING BONE BREAK, BONE FRACTURE, BONE DEGENERATION AND OSTEOPOROSIS**
CCL5 ENTHALTENDE ZUSAMMENSETZUNG ZUR VERWENDUNG BEI DER BEHANDLUNG VON KNOCHENBRUCH, KNOCHENFRAKTUREN, KNOCHENDEGENERATION UND OSTEOPOROSE
COMPOSITION COMPRENANT CCL5 POUR UTILISATION DANS LE TRAITEMENT DE LA RUPTURE OSSEUSE, DE LA FRACTURE OSSEUSE, DE LA DÉGÉNÉRESCENCE OSSEUSE ET DE L'OSTÉOPOROSE

(30) Priority: 07.02.2019 US 201962802616 P
(43) Date of publication of application: 15.12.2021
(73) Proprietor: Baylor College of Medicine, Houston, TX 77030 (US)
(72) Inventor: PARK, Dongsu, Houston, Texas 77030 (US); ORTINAU, Laura, Houston, 77030 (US)
(74) Representative: J A Kemp LLP
(86) International application number: PCT/US2020/017301
(87) International publication number: WO 2020/163766

(56) References cited:
- US-A1- 2004 191 221
- US-A1- 2010 239 634
- US-A1- 2011 182 904
- US-A1- 2011 182 904
- US-A1- 2015 359 852
- US-A1- 2019 015 448
- LI B ET AL: "The effects of rhBMP-2 released from biodegradable polyurethane/microsphere composite scaffolds on new bone formation in rat femora", BIOMATERIALS, ELSEVIER, AMSTERDAM, NL, vol. 30, no. 35, 17 September 2009 (2009-09-17), pages 6768 - 6779, XP026693730, ISSN: 0142-9612, [retrieved on 20090917], DOI: 10.1016/J.BIOMATERIALS.2009.08.038
- YU Y Y ET AL: "Bone morphogenetic protein 2 stimulates endochondral ossification by regulating periosteal cell fate during bone repair", BONE, PERGAMON PRESS., OXFORD, GB, vol. 47, no. 1, 27 March 2010 (2010-03-27), pages 65 - 73, XP027074957, ISSN: 8756-3282, [retrieved on 20100603], DOI: 10.1016/J.BONE.2010.03.012
- STEVEN MINEAR ET AL: "Wnt Proteins Promote Bone Regeneration", SCIENCE TRANSLATIONAL MEDICINE, 28 April 2010 (2010-04-28), United States, pages 29ra30 - 29ra30, XP055449646, Retrieved from the Internet <URL:https://www.science.org/doi/pdf/10.1126/scitranslmed.3000231> DOI: 10.1126/scitranslmed.3000231
- G. E. GLASS ET AL: "TNF- promotes fracture repair by augmenting the recruitment and differentiation of muscle-derived stromal cells", PROCEEDINGS OF THE NATIONAL ACADEMY OF SCIENCES, vol. 108, no. 4, 5 January 2011 (2011-01-05), pages 1585 - 1590, XP055729838, ISSN: 0027-8424, DOI: 10.1073/pnas.1018501108
- JI-WON LEE ET AL: "The HIV co-receptor CCR5 regulates osteoclast function", NATURE COMMUNICATIONS, vol. 8, no. 1, 20 December 2017 (2017-12-20), XP055729839, DOI: 10.1038/s41467-017-02368-5
- ORTINAU LAURA ET AL: "Periosteal skeletal stem cells are a functionally and genetically distinct subset of skeletal stem cells necessary for bone healing", JOURNAL OF BONE AND MINERAL RESEARCH, vol. 33, 16 November 2018 (2018-11-16), US, pages 6 - 6, XP055972679, ISSN: 0884-0431, Retrieved from the Internet <URL:https://onlinelibrary.wiley.com/doi/pdf/10.1002/jbmr.3621> DOI: 10.1002/jbmr.3621
- WINTGES KRISTOFER ET AL: "Impaired bone formation and increased osteoclastogenesis in mice lacking chemokine (C-C motif) ligand 5 (Ccl5)", JOURNAL OF BONE AND MINERAL RESEARCH, vol. 28, no. 10, 2 April 2013 (2013-04-02), US, pages 2070 - 2080, XP055972616, ISSN: 0884-0431, DOI: 10.1002/jbmr.1937
- ANGIOLILLO A L ET AL: "HUMAN INTERFERON-INDUCIBLE PROTEIN 10 IS A POTENT INHIBITOR OF ANGIOGENESIS IN VIVO", JOURNAL OF EXPERIMENTAL MEDICINE, ROCKEFELLER UNIVERSITY PRESS, US, vol. 182, 1 July 1995 (1995-07-01), pages 155 - 162, XP002916077, ISSN: 0022-1007, DOI: 10.1084/JEM.182.1.155
- KHOSROW S. HOUSCHYAR ET AL: "Wnt Pathway in Bone Repair and Regeneration - What Do We Know So Far", FRONTIERS IN CELL AND DEVELOPMENTAL BIOLOGY, vol. 6, 7 January 2019 (2019-01-07), XP055728463, DOI: 10.3389/fcell.2018.00170
- YU , YY ET AL.: "Bone morphogenetic protein 2 stimulates endochondral ossification by regulating periosteal cell fate during bone repair", BONE, vol. 47, no. 1, 27 March 2010 (2010-03-27), pages 1 - 18, XP027074957
- GLASS, GE ET AL.: "TNF-alpha promotes fracture repair by augmenting the recruitment and differentiation of muscle-derived stromal cells", PROCEEDINGS OF THE NATIONAL ACADEMY OF SCIENCES OF THE USA, vol. 108, no. 4, 5 January 2011 (2011-01-05), pages 1585 - 1590, XP055729838
- LEE, JW ET AL.: "The HIV co-receptor CCR5 regulates osteoclast function", NATURE COMMUNICATIONS, vol. 8, no. 2226, 20 December 2017 (2017-12-20), pages 1 - 16, XP055729839

## Description

### TECHNICAL FIELD

Aspects of the disclosure include at least the fields of molecular biology, cell biology, genetics, and medicine.

### BACKGROUND

Stem cells are indispensable for the repair of most tissue injuries through distinct regulatory mechanisms (Xin et al., 2016), and dysregulation of these cells often leads to impaired/delayed healing (Riminucci et al., 2015). In particular, multipotent skeletal stem cells (SSCs) are known to exist within the bone marrow (BM) and provide mature osteoblasts, chondrocytes, adipocytes, and marrow stromal cells required for skeletal development, homeostasis, and repair (Bianco and Robey, 2015). More recently, SSC populations have been identified within tissues other than BM (*e.g*. calvarial sutures and the periosteum), indicating that endogenous SSCs have multiple tissue locations. Yet, which SSCs recognize tissue injury and how they initiate the repair process remains a fundamental question.

The existence of diverse stem cell pools characterized by distinct molecular markers and context-dependent functions has been reported in multiple tissues (Goodell et al., 2015). Tissue-resident SSCs are also known to be a heterogeneous population with different locations. Long-term repopulating SSCs that contribute to bone regeneration and repair have been identified within the BM (Caplan, 1991; Friedenstein et al., 1974; Morikawa et al., 2009) (Chan et al., 2009; Chan et al., 2015), and they are a subset of BM perivascular cells marked by *Cxcl12*-DTR-GFP (Omatsu et al., 2010), *Nestin-*GFP (Mendez-Ferrer et al., 2010), *Prx1-*Cre (Greenbaum et al., 2013), *LepR-*Cre (Zhou et al., 2014), or *Mxl-*Cre (Park et al., 2012a). Interestingly, a non-perivascular SSC population with *in vivo* osteogenic and chondrogenic potential that is distinctly different from perivascular SSCs is labeled by Gremlin 1 *(Grem1)* (Worthley et al., 2015). Similar to BM, osteo/chondrogenic stem/progenitors have also been found in the periosteum and are reported to have an important role in fracture repair (Roberts et al., 2015). These Prx1- or Axin2- expressing periosteal progenitors contribute to outer bone shaping, cortical thickness, and fracture repair (Ouyang et al., 2013; Ransom et al., 2016; Roberts et al., 2015; Wilk et al., 2017). In addition, these periosteal cells are particularly important in the life-long repair of bones with limited or no BM, suggesting that SSCs reside in the periosteum.

Fracture repair is a dynamic multi-cellular process distinct from embryonic skeleton development and remodeling. During this process, the rapid recruitment, proliferation, and subsequent differentiation of skeletal stem/progenitor cells are critical for overall stability and fixation of fractures (Einhorn and Gerstenfeld, 2015). Multiple cytokines and growth factors such as Wnt, FGF2, PDGF, and BMPs are known to regulate bone remodeling and repair and have been tested for the enhancement of skeletal repair in critical segment defects (Einhorn and Gerstenfeld, 2015; Schindeler et al., 2008). However, because of the lack of specific markers to distinguish SSCs at different locations and the cellular complexity of injury models generated through disruption of the bone compartment's physical barrier, the direct effect of these molecules on endogenous SSCs during injury healing is unclear. New animal models have been developed that allow real-time tracking of the recruitment and activation of endogenous SSCs at different locations, and sought to identify the origin and unique characteristics of periosteum-resident SSCs (P-SSCs) responsible for cortical bone healing. However, the ability to specifically recruit such cells to a specific site is needed in the art, as is the need for bone repair treatments.

Li et al. (Biomaterials, vol. 30, no. 35, 1 December 2009, pages 6768-6779) discloses the effects of rhBMP-2 released from biodegradable polyurethane/microsphere composite scaffolds on new bone formation in rat femora. Yu et al. (Bone, vol. 47, no. 1, 1 July 2010, pages 65-7) discloses that bone morphogenetic protein 2 stimulates endochondral ossification by regulating periosteal cell fate during bone repair. Minear et al. (Science Translational Medicine, 28 April 2010, pages 29ra30-29ra30) discloses that Wnt Proteins Promote Bone Regeneration. Glass et al. (PNAS, vol. 108, no. 4, 5 January 2011, pages 1585-1590,) discloses that TNFα; promotes fracture repair by augmenting the recruitment and differentiation of muscle-derived stromal cells. US 2011/182904 discloses isolated monoclonal antibodies, particularly human monoclonal antibodies, which bind to BMP2, BMP4, BMPR1A, BMPR1B, ACTR1, and/or BMPR2. Angiolillo et al. (Journal Of Experimental Medicine, vol. 182, 1 July 1995, pages 155-162) discloses that human interferon-inducible protein 10 is a potent inhibitor of angiogenesis *in vivo.*

### SUMMARY OF THE INVENTION

The invention provides a composition comprising CCL5 for use in method of treating bone break, bone fracture, bone degeneration or osteoporosis in an individual, the method comprising administering the composition to the individual at a site in need thereof.

Further aspects of the invention and preferred embodiments are defined in the dependent claims. Any aspects, embodiments and examples of the present disclosure which do not fall under the scope of the appended claims do not form part of the invention and are merely provided for illustrative purposes.

### OVERVIEW OF THE DISCLOSURE

The present disclosure is directed to systems, methods, and compositions for administering to a specific site in an individual in need. In specific aspects of the disclosure, the site is one or more bone sites. The site in need may be a bone break, bone fracture, bone degeneration, site of osteoporosis, site of osteopetrosis, site of osteosclerosis, or other bone degenerative diseases, or a site that would benefit from prophylactic systems, methods, or compositions to delay or inhibit bone degeneration. The individual may also be administered one or more other conventional or novel methods and/or compositions to aid in the bone healing process. Such methods include, for example, surgery, placing the individual in a cast or sling, a fixation device, or a combination thereof. The site in need may be monitored for severity of need and also may be monitored for efficacy of the disclosed systems, methods, and/or compositions during and/or after the administration. One skilled in the art can determine the efficacy of the disclosed systems, methods, or compositions and adjust the dosage and timing of the administration in order to effectively induce bone healing in the individual.

In some aspects of the disclosure, there are methods of inducing bone healing in an individual comprising administering one or more cytokines to the individual at a site in need, including at a bone site. In particular aspects, the disclosure is directed to methods of inducing bone healing at least at one site in need in an individual. In specific aspects, the disclosure is directed to methods of recruiting periosteal skeletal stem cells (P-SSCs) to the site in need. The P-SSCs may differentiate into osteoblasts and aid in the induction of bone healing, in at least some cases. In specific aspects, the disclosure is directed to methods to reduce osteoclast migration to a site in need, wherein osteoclasts may reduce the induction of bone healing. Examples of the aspects provided may be achieved through the administration of at least one cytokine at the site in need of bone healing. The cytokine administered may be, in particular aspects, CCL5 and/or TNFα. In some aspects, stem cells, including for example P-SSCs, may be administered to an individual. The administered P-SSCs may express Mx1 and/or αSMA. The P-SSCs may be administered locally (such as at a site in need) and/or systemically.

In particular aspects, the disclosure is directed to methods of impairment of bone development at a specific site in an individual, for example wherein the recruitment of osteoclasts is desired. The method of impairing bone development may be achieved through the administration of one or more compositions that inhibit the function of CCR5, TNFα, or both. An individual may be in need of a reduction of bone healing or development, such as with one or more bone spurs or one or more sites of ectopic bone formation or one or more bunions, for example.

Administration of any composition encompassed by the disclosure may comprise injection or delivery of the cytokine(s), stem cells, or other composition(s) into the individual at the site in need. The cytokine or composition may be administered with a biocompatible gel or scaffold, which may allow for local cytokine signaling. The cytokine or composition may be formulated with a biocompatible gel or scaffold prior to administration, wherein the formulation is a new composition. The cytokine(s), cells, or composition(s) can be administered a single time or the cytokine(s), cells, or composition(s) can be administered multiple times, wherein the number of administrations needed is assessed by one skilled in the art.

Described herein are methods of inducing bone healing in an individual comprising administering at least one cytokine to the individual at a site in need, wherein the cytokine is selected from the group consisting of CCL5, TNFα, BMP2, Wnt, or a combination thereof. For instance, the invention provides a composition comprising CCL5 for use in method of inducing bone healing treating bone break, bone fracture, bone degeneration, or osteoporosis in an individual, the method comprising administering the composition to the individual at a site in need thereof. In some cases, administering of the cytokine comprises delivery directly to the site in need (as an example, by injection), although in some cases it is not, such as being systemic. Administering may or may not comprise delivery of the cytokine with a gel or scaffold (for example, matrigel, hydrogel, periosteum, hydroxyapatite, tricalcium phosphate, polystyrene, poly-l-lactic acid, polyglycolic acid, poly-dl-lactic-co-glycolic acid, collagen, proteoglycans, alginate-based substrates, chitosan, collagen-GAG, extracellular matrix, or a combination thereof).

Administration of any composition of the disclosure may or may not comprise delivery of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 ng of cytokine to the site in need. Administration of any composition of the disclosure may or may not comprise delivery of at least about 100, 200, 300, 400, 500, 600, 700, 800, 900, 1000, 1100, 1200, 1300, 1400, 1500, 1600, 1700, 1800, 1900, 2000, 2500, 3000, 3500, 4000, 4500, 5000, 5500, 6000, 6500, 7000, 7500, 8000, 8500, 9000, 9500, 10000, or more stem cells. The administering of any composition may or may not comprise at least one delivery.

In particular cases, the delivery indirectly or directly provides the composition to a site in need, such as a bone fracture, bone break, bone degeneration, a site of osteoporosis, or a combination thereof. The site in need may or may not be monitored for bone regeneration.

In specific aspects of the disclosure, the administering comprises administration of at least one cytokine and at least one additional therapy for the site in need, such as surgical repair, fixation of a device to set the site in need, placing the individual in a cast or sling, or a combination thereof.

Aspects disclosure herein for illustrative purposes include methods to recruit periosteal skeletal stem cells to a site in an individual in need thereof, comprising administering at least one cytokine to the individual at a site in need, wherein the cytokine is selected from the group consisting of CCL5, TNFα, BMP2, Wnt, or a combination thereof.

Any individual treated with methods provided herein may or may not have osteopetrosis or osteosclerosis.

Particular aspects of the disclosure encompass methods to reduce osteoclast migration to a site in need in an individual comprising administering at least one cytokine to the individual at a site in need, wherein the cytokine is selected from the group consisting of CCL5, TNFα, BMP2, Wnt, or a combination thereof.

Aspects disclosed herein for illustrative purposes include methods for inhibiting bone formation at a site in need in an individual comprising administering at least one agent that inhibits the function of CCR5 TNFα, BMP2, or Wnt. The agent may or may not be maraviroc or an antibody. One example of bone formation to be inhibited is a bone spur or bunion. Administration of the agent may or may not comprise delivery directly to the site in need and may comprise delivery of the agent with a gel or scaffold. Administration may or may not comprise delivery of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 mM of agent to the site in need. Administration may comprise at least one delivery to the individual. In at least some cases, the site in need comprises a bone spur or ectopic bone formation site or bunion. The site in need may be monitored for the presence or absence of bone regeneration, or bone loss. In some aspects of the disclosure, the administration to the individual may be prophylactic.

Aspects disclosed herein for illustrative purposes include compositions for administering to a bone site comprising one or more cytokines and/or agents, and optionally one or more gels and/or scaffolds. In specific cases, the cytokine and/or agent comprises CCL5, TNFα, BMP2, Wnt, maraviroc, an antibody that targets CCL5, an antibody that targets TNFα, an antibody that targets BMP2, an antibody that targets Wnt, an antibody that targets CCR5 or a combination thereof. The gel and/or scaffold may or may not comprise matrigel, hydrogel, periosteum, hydroxyapatite, tricalcium phosphate, polystyrene, poly-l-lactic acid, polyglycolic acid, poly-dl-lactic-co-glycolic acid, collagen, proteoglycans, alginate-based substrates, chitosan, collagen-GAG, extracellular matrix, or a combination thereof.

The foregoing has outlined rather broadly the features and technical advantages of the present disclosure in order that the detailed description that follows may be better understood. Additional features and advantages will be described hereinafter which form the subject of the claims herein.

### BRIEF DESCRIPTION OF THE DRAWINGS

For a more complete understanding of the present disclosure, reference is now made to the following descriptions taken in conjunction with the accompanying drawings.

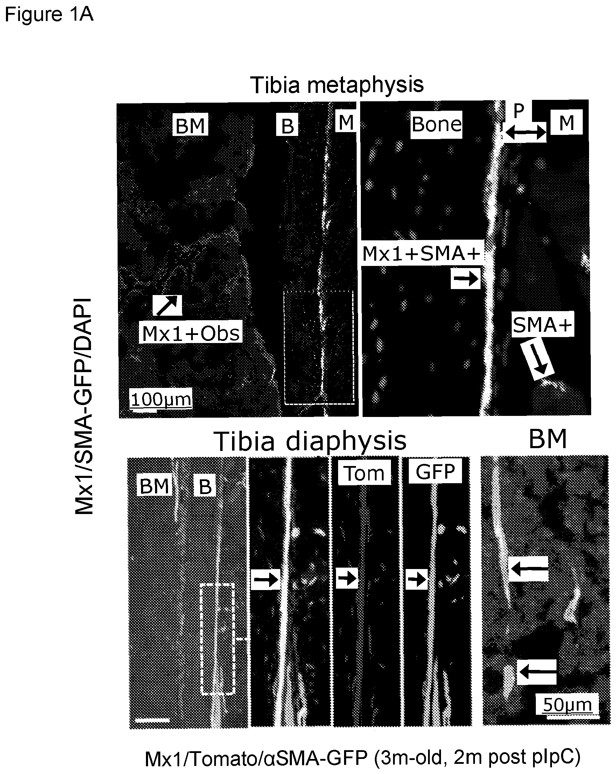

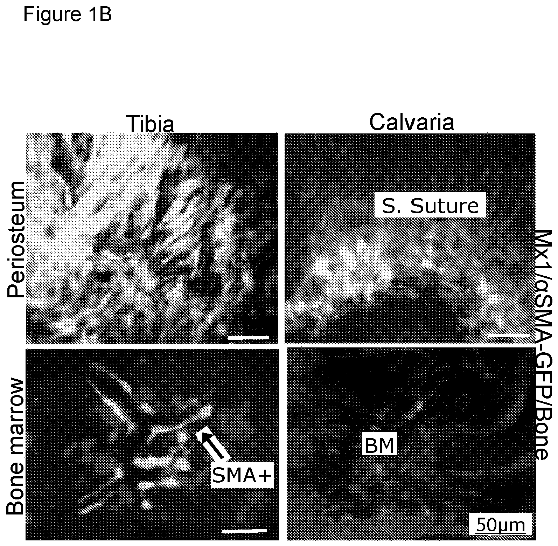

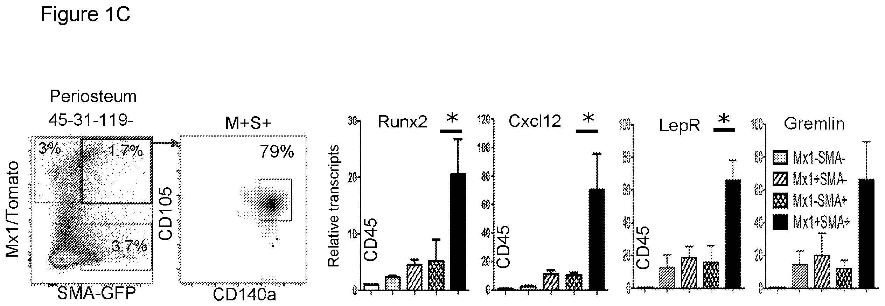

FIGS. 1A-1G show *Mx1⁺αSMA⁺* periosteal cells highly express SSPC markers and contribute to bone injury repair. *Mx1*-Cre activity was induced at 4-weeks of age in Mx1/Tomato/ αSMA-GFP mice with 5 doses (10 days) of pIpC. Two-month old Mx1/Tomato/αSMA-GFP dual reporter mice were lethally irradiated and received wild type-bone marrow transplant (10⁶ cells) to reduce the background of hematopoietic lineage cells. **FIGS. 1A** **&** **1B****.** The periosteal *Mx1*⁺*αSMA*⁺ population in the tibia and calvaria suture of Mx1/Tomato/SMA-GFP dual reporter mice was analyzed by immunofluorescence staining **(****FIG. 1A****)** and intravital microscopy **(****FIG. 1B****),** respectively. Arrows indicate *Mx1*⁺*αSMA*⁺ cells (red and green) in the periosteum **(P,** **FIG. 1A****)** and suture **(****FIG. 1B****),** *Mx1*⁺*αSMA⁻* osteoblasts (Mx1⁺ Obs, red) in the endosteum, and *αSMA⁺* cells (SMA+, green) in muscle. M: muscle; B: bone; BM: bone marrow. **FIG. 1C****.** High expression of SSPC surface markers and transcription factors in the *Mr1⁺αSMA*⁺ fraction in the periosteum. Graph shows percentages of SSPC surface markers (CD105⁺ and CD140a⁺) and relative expression of SSPC transcription factors (Runx2, Cxcl12, LepR, & Gremlin) from periosteal *Mx1*⁺*αSMA*⁺ cells in comparison to those of *Mx1⁺αSMA⁻, Mx1⁻αSMA⁺, Mx1⁻αSMA⁻, or CD45⁺* cells from the periosteum determined by flow cytometry (n=6). **1D.** A representative single-cell-driven hemisphere colony formation of *Mx1*⁺*αSMA*⁺ periosteal cells and their osteogenic, adipogenic, and chondrogenic potential. Alizalin red, oil red O, and Toluidine-blue staining were used for osteogenic, adipogenic, chondrogenic differentiation, respectively. **1E.** Injury-mediated expansion of the *Mx1*⁺ and *Mx1*⁺*αSMA*⁺ populations (red and white arrows) in the callus and new periosteum (New PO, red arrow), but not in bone marrow (BM), supply the majority of the new bone-forming osteoblasts (new bone) two weeks post-injury (representative of 5 independent experiments). *Mx1*⁺*αSMA*⁺ (red and green) cells and their contribution to new chondrocytes (left, arrows) and osteoblasts (right, arrows) were assessed by immunostaining of the injury sites with anti-aggrecan antibody (left, white) and anti-osteocalcin antibody (right, white). **1F.** After calvaria injuries in *Mx1*/tomato/αSMA-GFP dual reporter mice at 4 months of age, *Mx1*⁺*αSMA*⁺ (yellow) periosteal cells (D0 injury) and their repopulation at injury sites (D21 injury) were assessed by sequential *in vivo* imaging. Ob, osteoblast. **1G.** 3-D reconstruction of *in vivo* z-stack images at twenty-one days post-injury of the calvaria show that *Mx1⁺αSMA⁺* (red and green) cells remain undifferentiated in periosteum (left) and in the middle of injury (right, yellow), while they become *Mx1⁺αSMA⁻* osteoblasts (layer a, arrow) near bone surface and/or osteocytes (layer b, arrow) in new bone (blue).

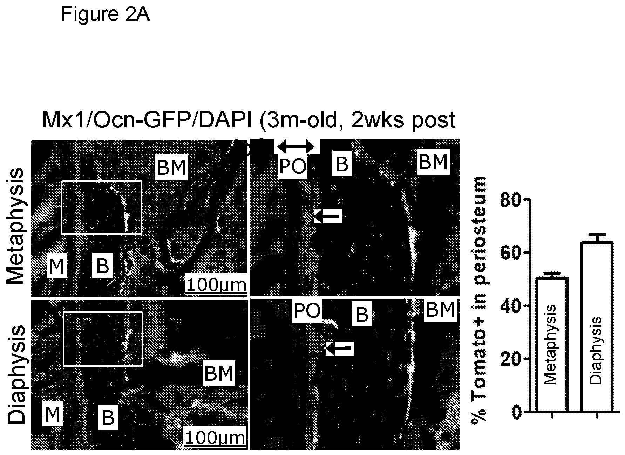

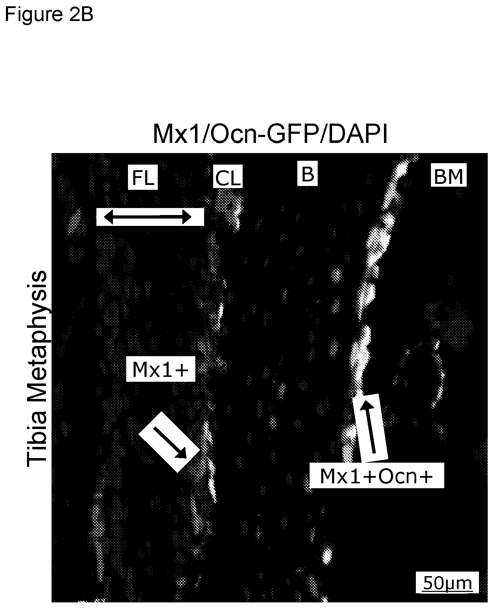

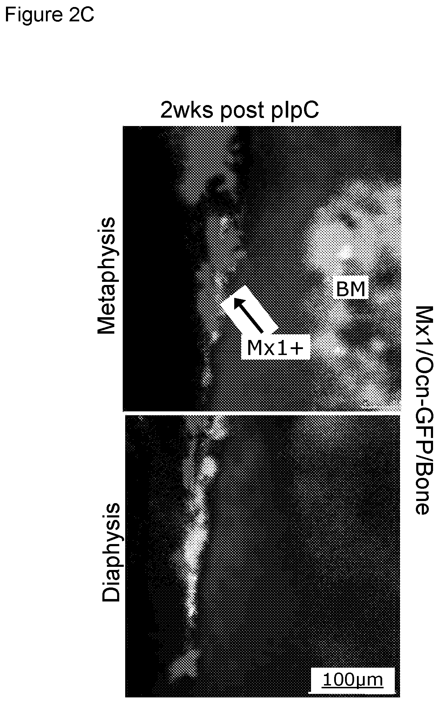

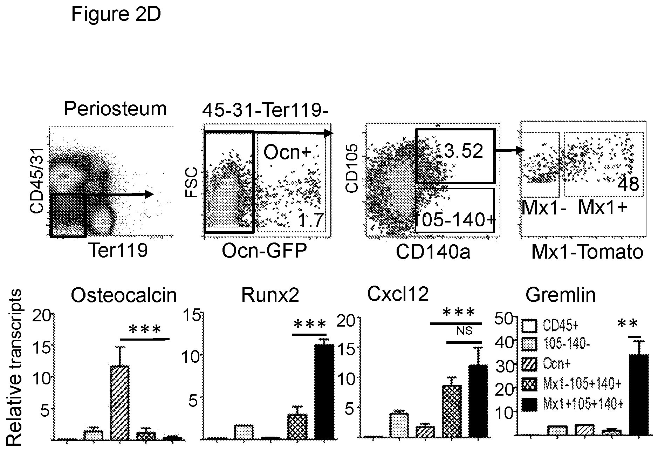

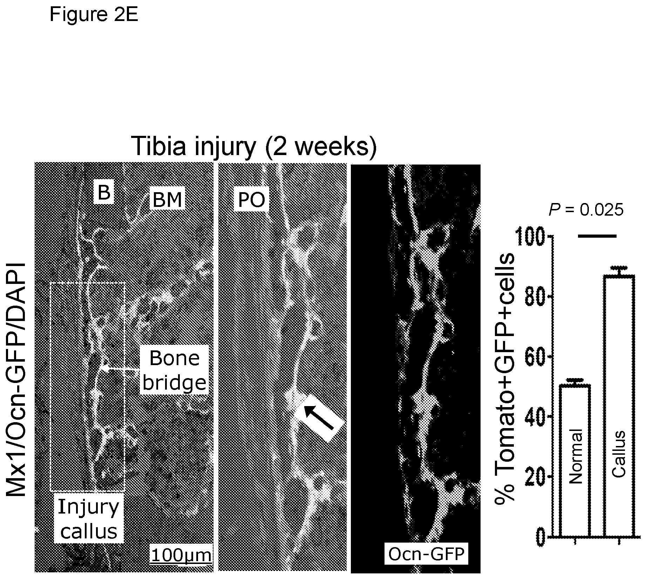

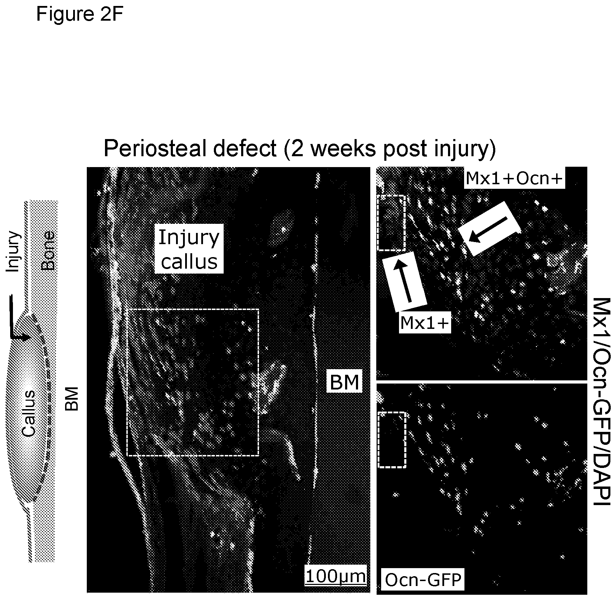

FIGS. 2A-2F shows *Mx1*⁺ osteogenic progenitors in the periosteum supply the majority of osteoblasts in cortical bone injury. Four-week-old Mx1/Tomato/Ocn-GFP dual reporter mice were lethally irradiated and received wild type-bone marrow transplant (10⁶ cells) to reduce the background of hematopoietic lineage cells. *Mx1*-Cre activity was induced at 2-months of age in Mx1/Tomato/Ocn-GFP mice with 5 doses (10 days) of pIpC. **FIGS. 2A-2C****.** Periosteal *Mx1*⁺ progenitor cells (Mx1+: red, arrows) and endosteal osteoblasts (green) and new osteoblasts from *Mx1*⁺ progenitor cells (Mx1+Ocn+: red and green) in the tibial metaphysis and diaphysis of Mx1/Tomato/Ocn-GFP mice were analyzed by immunofluorescent staining **(****FIGS. 2A** and **2B****)** and intravital imaging **(****FIG. 2C****).** Graph in A shows percentage of *Mx1*⁺ (Tomato+) progenitor cells in the periosteum of metaphysis and diaphysis (n=5). PO: periosteum; M: muscle; B: bone; BM: bone marrow; FL: fibrous layer; CL: Cambium layer. **FIG. 2D****.** *Mx1*⁺ progenitors in the periosteum show SSC characteristics. Hematopoietic cells (CD45⁺) and non-hematopoietic cells (CD45⁻CD31⁻Ter119⁻): bone marrow stromal cells (CD105⁻CD140⁺), osteoblasts (Ocn⁺), *Mx1⁻* (CD105⁺CD140⁺*Mx1*⁻), and *Mx1*⁺ (CD105⁺CD140⁺*Mx1*⁺) SSCs from Mx1/Tomato/Ocn-GFP mouse bones were sorted, and the levels of the indicated genes were quantified by qPCR. ** *P*<0.01; ****P<*0.001. **FIGS. 2E** **&** **2F****.** Representative tibia drill-hole defect **(****FIG. 2E****)** or periosteal defect **(****FIG. 2F****,** defect on cortical bone surface) showing that *Mx1*⁺ periosteal progenitors (red) contribute the majority of callus-forming osteoblasts (Mx1+Ocn+) in the injury callus (left box in **FIG. 2F****)** 2 weeks after injury. Expansion of *Mx1*⁺ progenitors (red) in new periosteum (PO) at the injury site is indicated by the arrows (arrow in **FIG. 2E** middle and white box in **FIG. 2F** top). Graph shows percentage of *Mx1⁺Ocn⁺* (Tomato+ and GFP+) osteoblasts from uninjured endosteum (normal) and injured bone callus (Callus) (n=5).

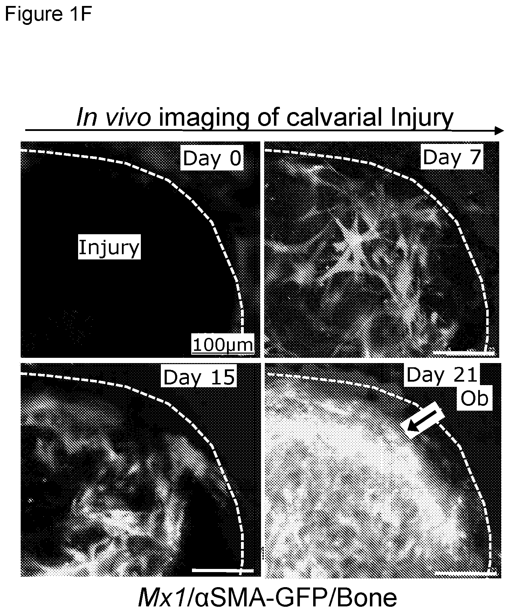

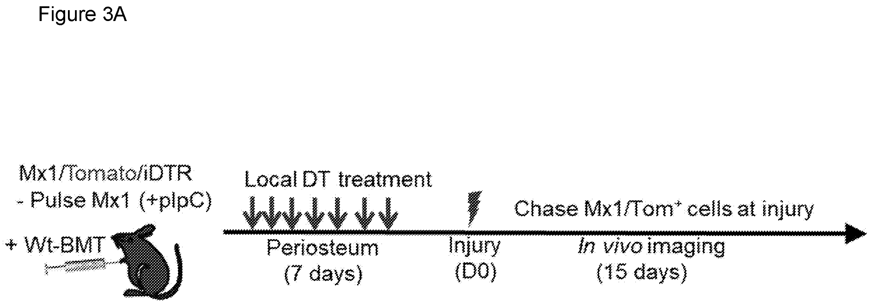

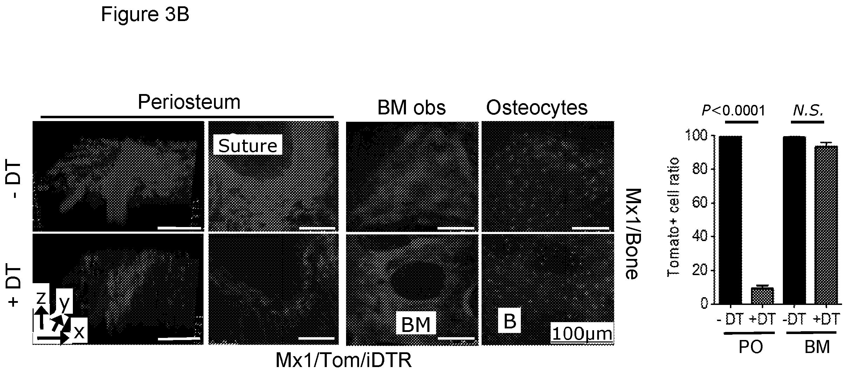

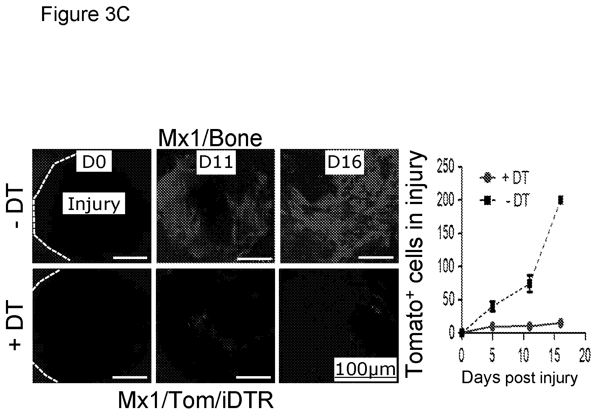

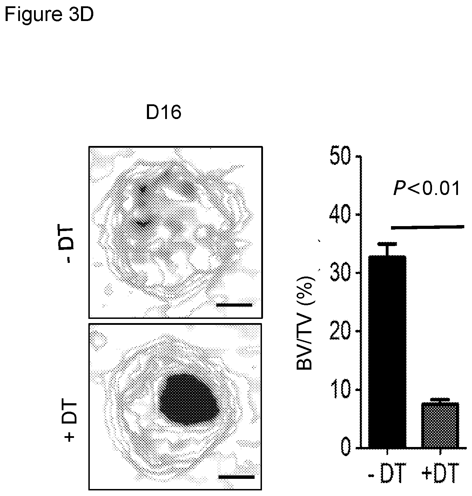

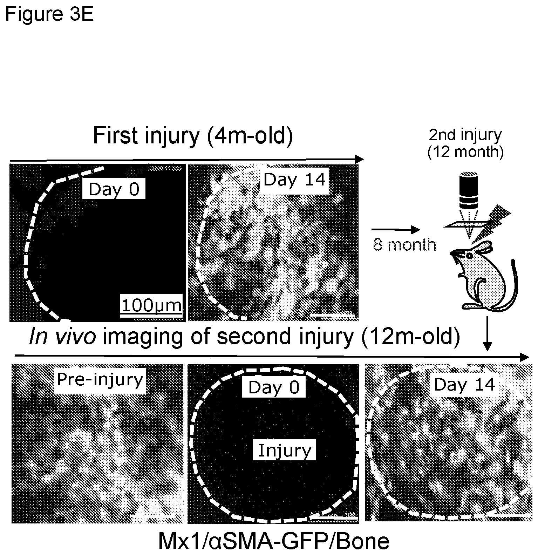

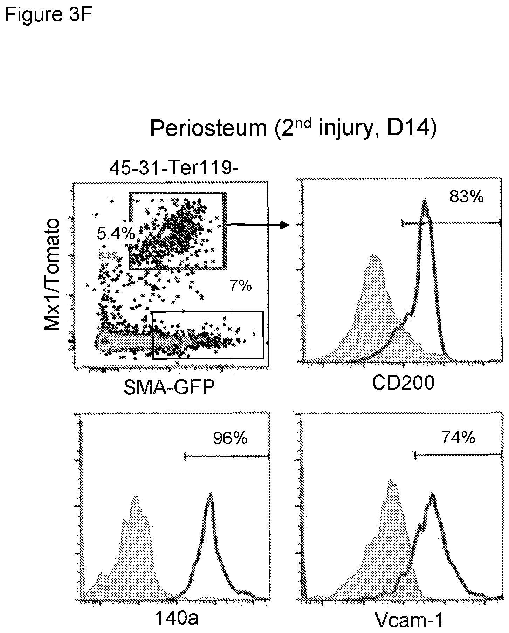

FIGS. 3A-3F show *Mr1*⁺ P-SSCs are necessary for bone healing and remain in the periosteum after injury. **FIG. 3A****.** The periosteum of *Mx1*/tomato/iDTR mice were treated locally with a low-dose of ditheria toxin (+DT; 20 µL at 1 µg/mL) or control (-DT; PBS) for seven days to ablate *Mx1*⁺ (tomato⁺) periosteal cells, but not bone marrow cells. **FIG. 3B****.** *In vivo* imaging before injury, the DT-mediated reduction of the *Mx1*⁺ (red) populations in the periosteum (PO) without change of *Mx1*⁺ bone marrow (BM) resident osteoblasts (BM obs) or osteocytes within the bone (B, blue) was analyzed. **FIG. 3C****.** After injury, the numbers of *Mx1*⁺ (red) cells at the calvarial injury sites were tracked at the indicated time points using *in vivo* imaging of the bone injury. Local ablation of *Mx1*⁺ periosteal cells prevents osteoprogenitor/osteoblast expansion at the injury sites, resulting in a significant reduction in bone injury repair. **FIG. 3D****.** Calvarial bone healing of *Mx1*/tomato/iDTR mice treated with DT (+DT) or PBS (-DT) was analyzed by µCT (bone volume/total volume; BV/TV) at day 16. Data represent at least three independent experiments. Scale bars are 100 µm. **FIG. 3E****.** After calvaria injuries in *Mx1*/tomato/SMA-GFP dual reporter mice at 4 months of age (3 months after pIpC) (FIG. 1F), long-term maintenance of *Mx1*⁺*αSMA*⁺ P-SSCs (Tomato⁺GFP⁺ yellow, Pre-injury) and their repopulation at injury sites (D14 injury) were assessed by sequential *in vivo* imaging of a second round of injuries at 12 months of age (8 months after the first injury). Bone (blue) (n=6). **FIG. 3F****.** High expression SSPC markers are maintained in the *Mx1*⁺*αSMA*⁺ fraction in the periosteum after a second injury. Histograms show percentages of the CD200⁺, CD140a⁺, and Vcam-1⁺ SSC marker expression within *Mr1⁺αSMA⁺* P-SSCs (red) in comparison to those of *SMA-GFP⁺* cells (gray) from the periosteum determined by flow cytometry (n=2).

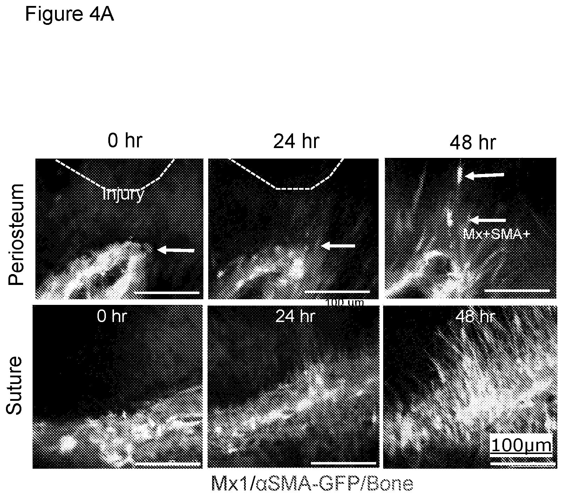

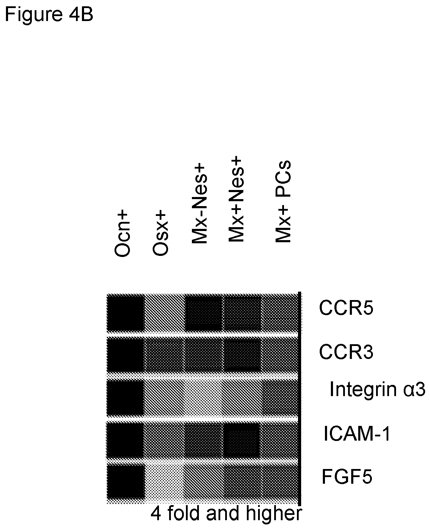

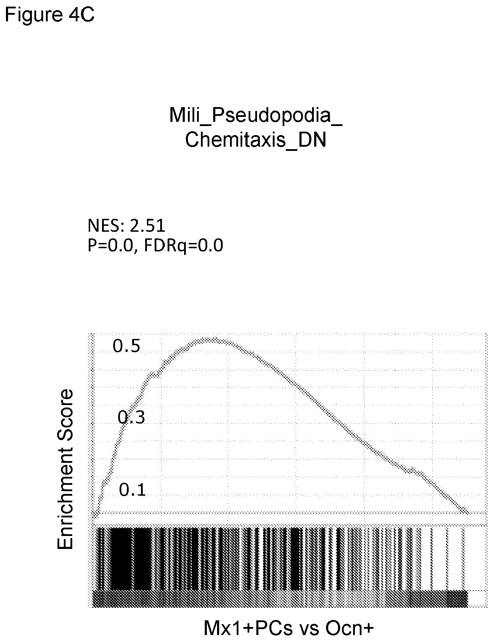

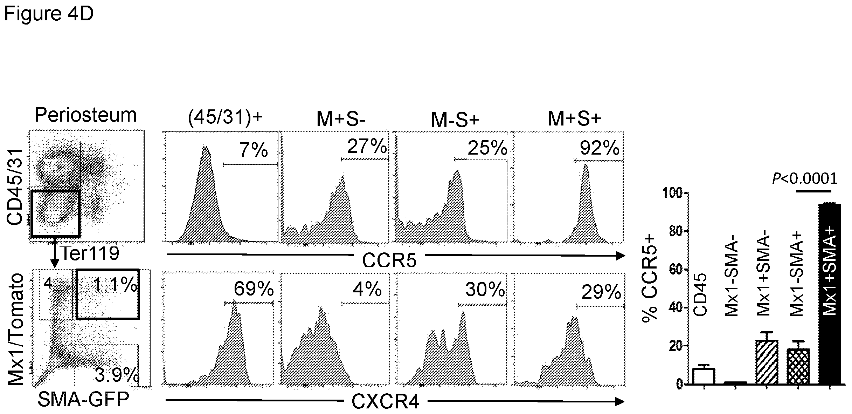

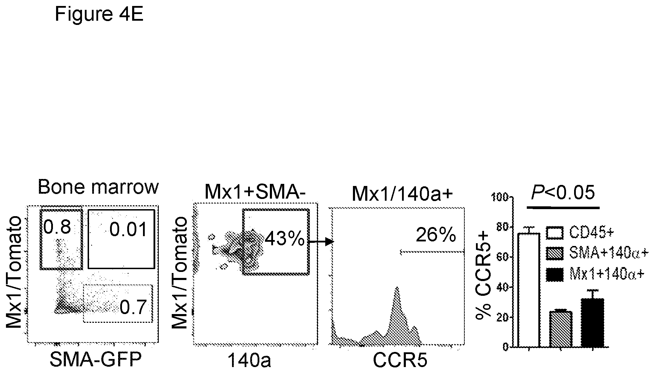

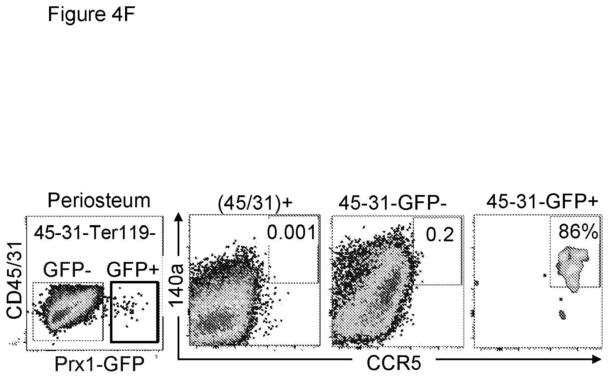

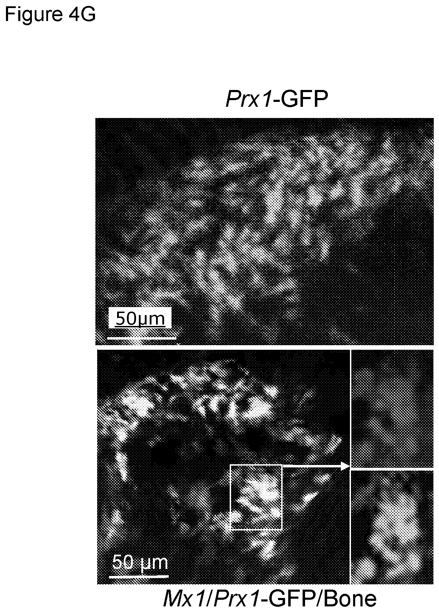

FIGS. 4A-4G show rapidly migrating *Mx1*⁺*αSMA*⁺ P-SSCs uniquely express CCR5 *in vivo.* **FIG. 4A****.** Using *Mxl*/*Tomato*/*SMA-GFP* dual reporter mice (3-month old, 2 months post pIpC from FIG. 1A), the early injury response of *Mx1*⁺*αSMA*⁺ periosteal cells (Periosteum) and suture mesenchymal cells (Suture) near the injury site was imaged at the indicated times after injury (≥ 5 replicates). Arrows indicate migrating *Mx1*⁺*αSMA*⁺ P-SSCs. Bone (blue), *αSMA⁺* cells (green), *Mx1*⁺ periosteal cells (red), *Mx1*⁺*αSMA*⁺ P-SSCs (red and green). Scale bar, 100 µm. **FIG. 4B****.** List of cell migration genes that are most abundantly expressed in *Mx1*⁺ periosteal cells (Mx1⁺ PCs) compared to control populations (FIG. 9D). **FIG. 4C****.** Chemotaxis gene set analysis of *Mx1*⁺ periosteal cells (Mx1+ PCs) vs. *osteocalcin-*GFP*⁺* osteoblasts (Ocn+ obs) (FIG. 9D). **FIG. 4D****.** Relative cell surface expression of CCR5 (top histograms, graph) and CXCR4 (bottom histograms) in *Mx1⁺αSMA⁺*P-SSCs (M+S+) from 3-4-month-old *Mx1*/*Tomato*/*SMA-GFP* dual reporter mice, compared to those of indicated cells and CD45⁺ cells, was analyzed by flow cytometry. **FIG. 4E****.** Relative cell surface expression of CCR5 in CD45⁺, Mx1⁺CD140a⁺, and αSMA⁺CD140a⁺ bone marrow cells. **FIG. 4F****.** Relative cell surface expression of CCR5 and CD140a in control CD45⁺CD31⁺ (45/31+) cells, 45⁻31⁻GFP⁻ stroma cells, or *Prx1*CreER-GFP⁺ cells from the periosteum of 1-month-old *Prx1*CreER-GFP⁺ mice. **FIG. 4G****.** *In vivo* imaging of periosteal *Prx1⁺* (green) population in the calvaria of *Prx1*CreER-GFP reporter mice without tamoxifen induction (top image). *Mx1*/tomato/*Prx1*-GFP mice were treated with 5 doses of pIpC every other day to induce *Mx1* labeling (red); followed by lethal irradiation and transplantation of wild-type BM. *In vivo* imaging of periosteal *Prx1⁺* (green) and *Mx1*⁺ (red) populations in the calvaria of *Mr1*/tomato/*Prx1*-GFP reporter mice without tamoxifen induction (bottom images). Box represents *Prx1⁺Mx1⁺* (red and green) cells.

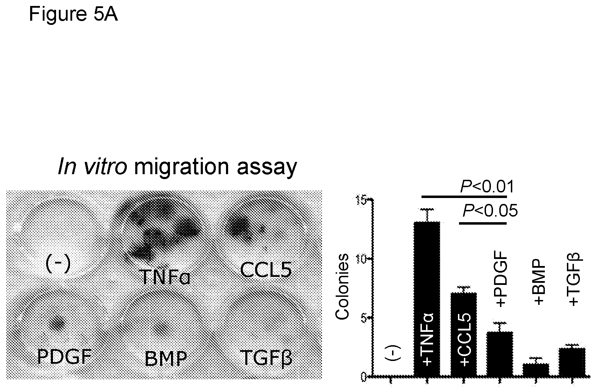

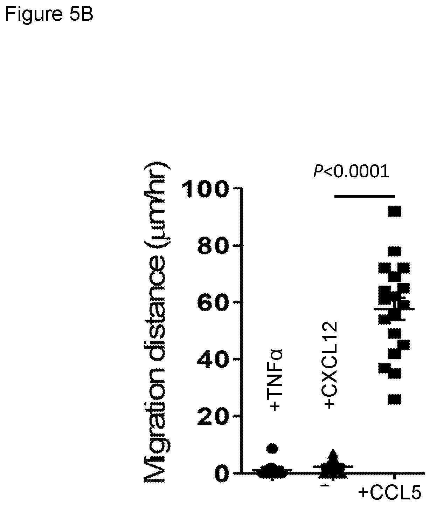

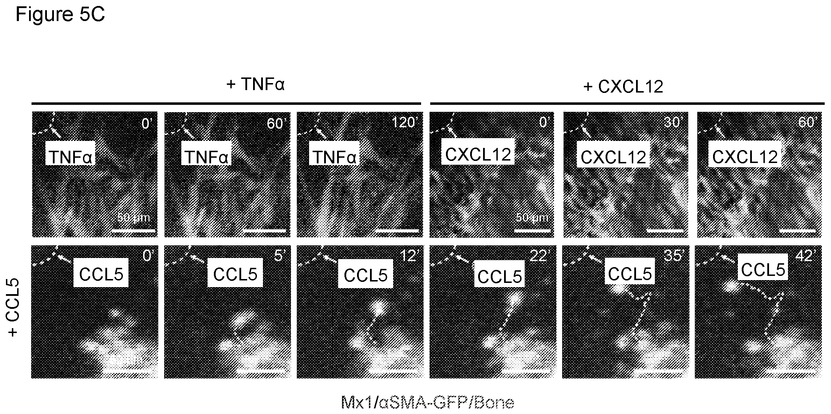

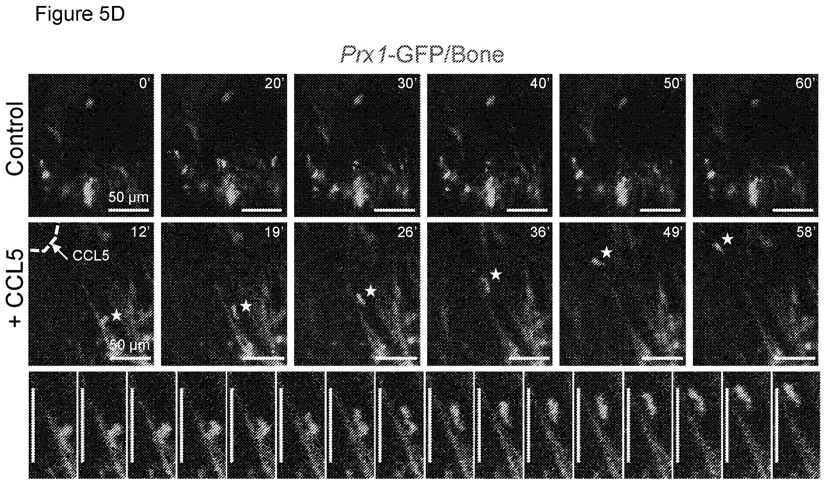

FIGS. 5A-5D shows the migration of P-SSC is induced by CCL5 *in vitro* and *in vivo.* **FIG. 5A****.** The effects of TNF-α (10 ng/mL), CCL5 (20 ng/mL), PDGF-β (20 ng/mL), BMP (20 ng/mL), and TGF-β (10 ng/mL) on FACS-sorted *Mx1*⁺P-SSCs (5,000/well) migration were examined, using a Transwell Assay (40-µm pore), for 12 hours, followed by their colony formation for 4 days. Relative migrating cell numbers from three independent experiments are shown on the right. **P*<0.05; ***P*<0.01. **FIGS. 5B** & **5C****.** *Mx1*/*Tomato*/*SMA-GFP* mice were treated with 5 doses (10 days) of pIpC to induce *Mx1* labeling (red); followed by lethal irradiation and transplantation of wild-type BM. Analysis were performed at least 1 month after WTBMT. **FIG. 5B****.** The average *in vivo* migration distance of individual cells induced by TNFα, CXCL12, or CCL5 was measured in *Mx1*/*Tomato*/*SMA-GFP* mice. **FIG. 5C****.** *In vivo* real-time imaging shows that CCL5, but not TNFα or CXCL12 (all 2 µL at 10 ng/µL of Matrigel), at the injury site induced the migration of *Mx1*⁺*αSMA*⁺ P-SSCs (≥3 replicates). The numbers indicate the time of imaging. Dotted line indicates the migratory path of *Mr1*⁺*αSMA*⁺ (yellow) cells. Scale bar for TNFα and CXCL12 treatment is 50 µm and CCL5 treatment is 20 µm. **FIG. 5D****.** CCL5 induced *in vivo* real-time migration of *Prx1⁺* (green) P-SSCs as assessed in *Prx1*CreER-GFP reporter mice treated with CCL5 (2 µL at 10 ng/µL of Matrigel) or Matrigel alone (2 µL, Control). Dotted line indicates migratory path of *Prx1⁺* (GFP⁺) P-SSCs. Bottom images represent sequential imaging of *Prx1⁺* P-SSC migration over 16 minutes (1 image per minute). Scale bar is 50 µm.

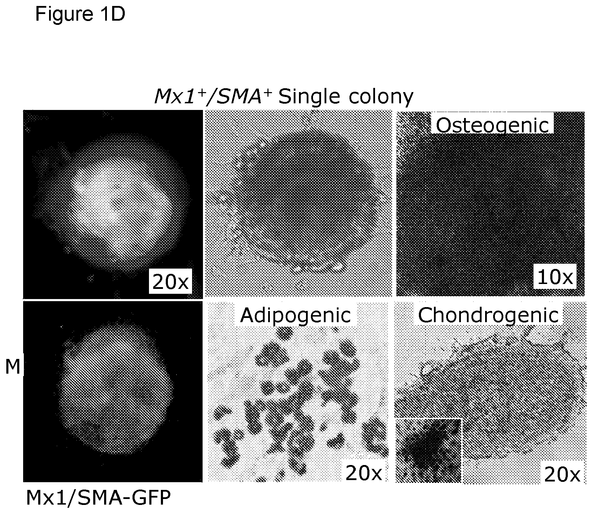

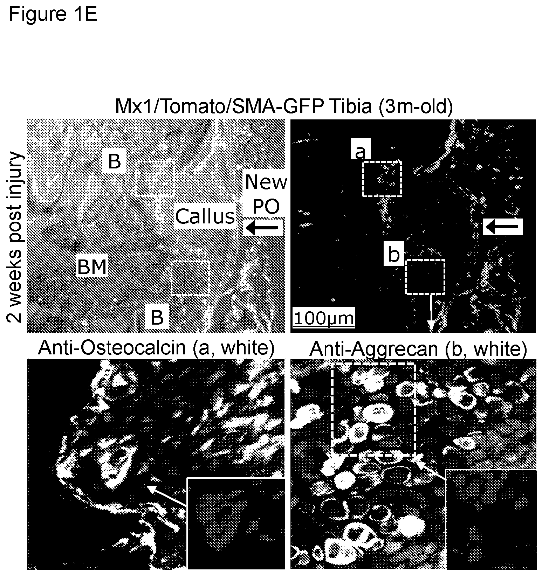

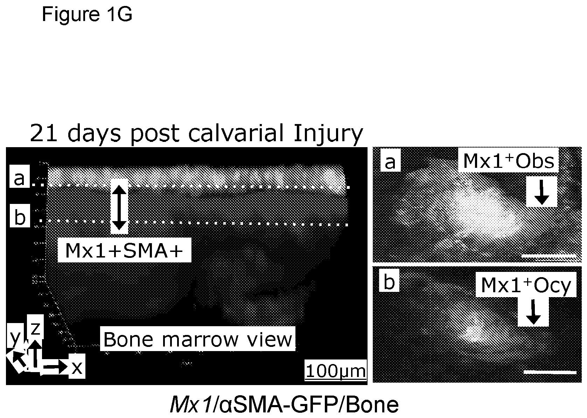

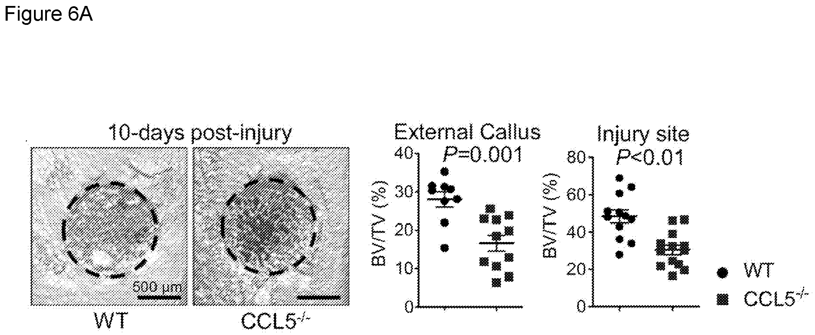

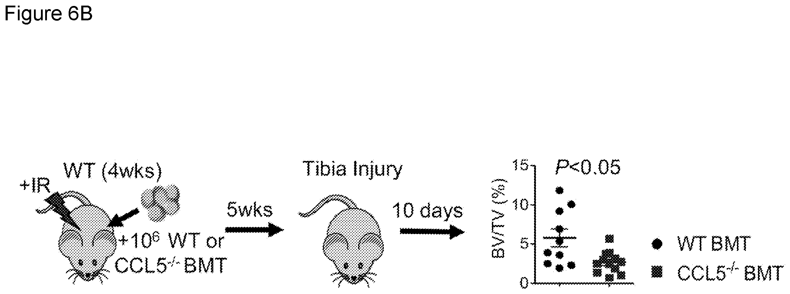

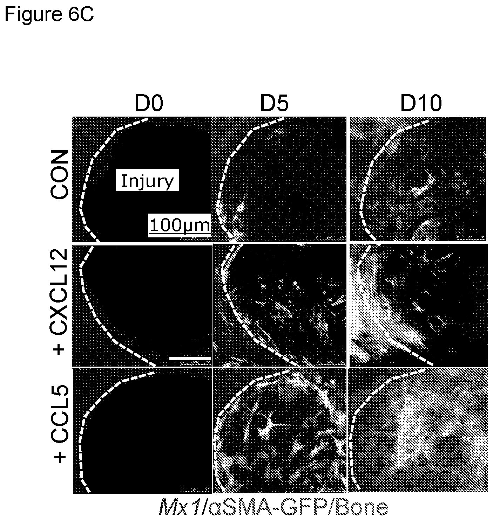

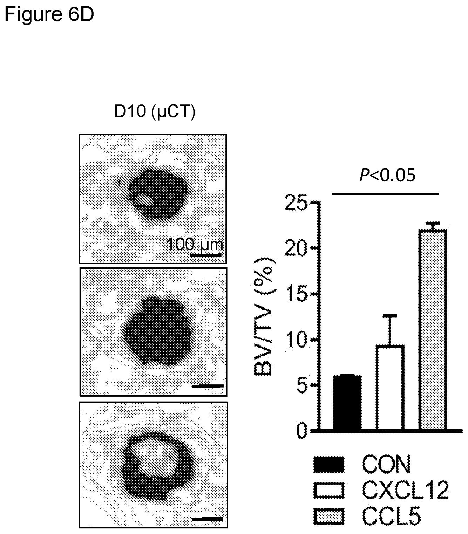

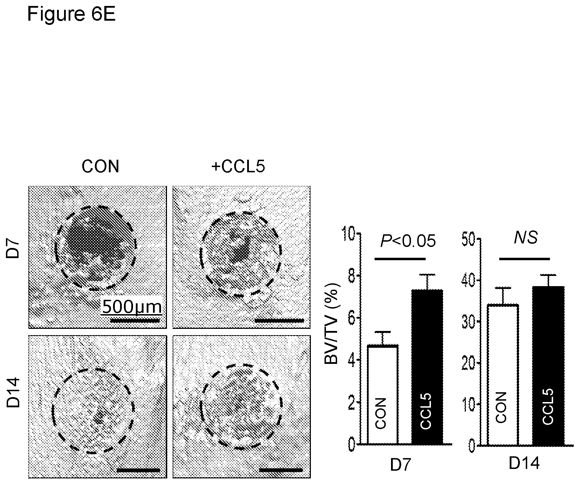

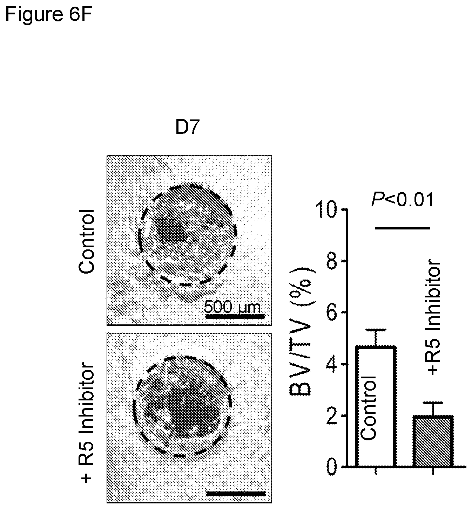

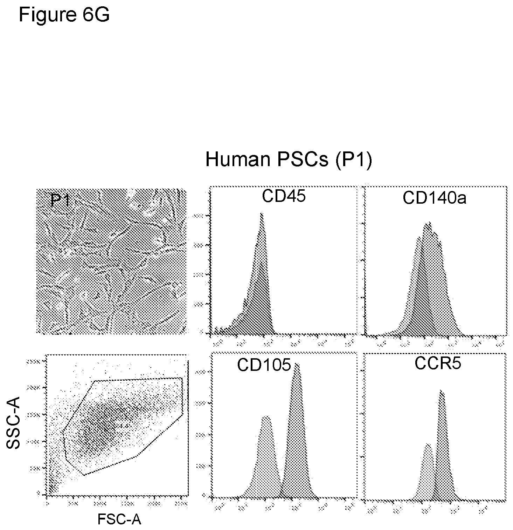

FIGS. 6A-6F show CCL5 treatment following bone injury induces P-SSC recruitment and activation leading to accelerated bone healing. **FIG. 6A****.** Representative µCT images of proximal tibial injuries (left) of age- and sex-matched WT (n=12) or 2- to 4- monthold Ccl5^{-/-} mice 10 days post injury (n=13). BV/TV of external callus (left) and injury sites (right) was assessed. **FIG. 6B****.** WT-BL6 mice (n = 10 per group) were irradiated (9.5 Gy) and transplanted with 106 WT (WT-BMT) or Ccl5^{-/-} BM mononuclear cells (Ccl5^{-/-} BMT). Five weeks later, BV/TV of injury sites was assessed at 10 days after tibial drill-hole injuries. **FIG. 6C****.** Sequential imaging of 3-4-month-old *Mxl*/*Tomato*/*αSMA-GFP* dual reporter mice prepared from FIG 1 and treated with supplemental CCL5 (2 µL at 10 ng/µL of Matrigel), CXCL12 (2 µL at 10 ng/µL of Matrigel), or or Matrigel alone (2 µL, con) at the indicated times post-injury. White dotted line indicates original injury sight; white bar is 100 µm. **FIG. 6D****.** Representative µCT images of calvaria injuries (from 6A) treated with CCL5, or CXCL12, ten days post-injury (left). Bone volume/total volume (BV/TV) was assessed for the quantification of bone healing (right). **FIG. 6E****.** Representative µCT images of proximal tibia injuries (left) treated with Matrigel (2 µL Matrigel; CON) or CCL5 (2 µL at 10 ng/µL of Matrigel; +CCL5) on Day 0, 2, and 4 post-injuryThe CCR5 inhibitor was injected (10 µL at 2.5 µg/µL in Matrigel) locally for 7 days post-injury. BV/TV was used to determine the effects of CCL5 or CCR5 inhibitor treatment on bone healing (right). **FIG. 6F****.** Representative µCT images of tibia injury treated locally with Matrigel (2 µL Matrigel; Control) or a CCR5 inhibitor (10 µL at 2.5 µg/µL in Matrigel; +R5 Inhibitor) on day 0, 2, 4 post injury. On Day 7 post-injury, subsequent bone volume/total volume (BV/TV) analysis was used for the quantification of bone healing (right). **FIG. 6G****.** Single-cell driven colonies from human periosteal tissue were cultured for 7-10 days (P1), then analyzed for expression of CCR5 and SSC markers (CD105 and CD140a). Isotype controls were used as negative controls (blue).

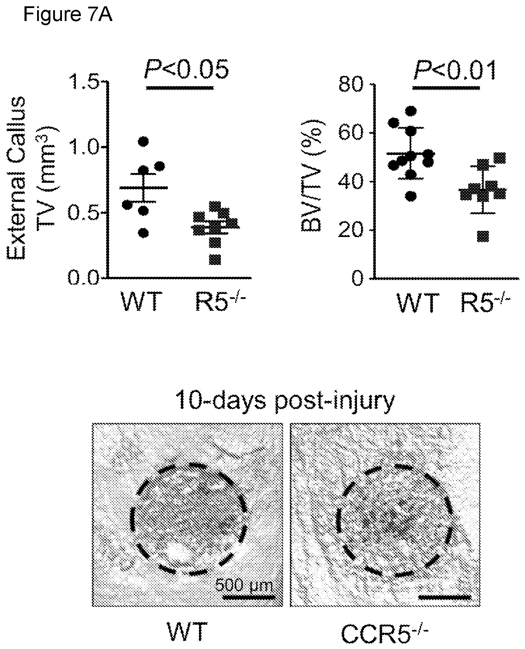

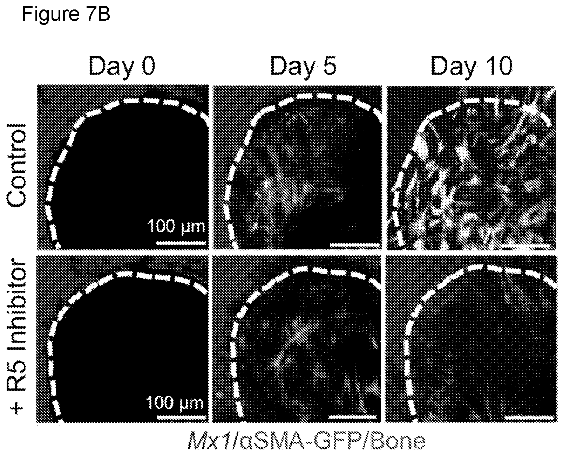

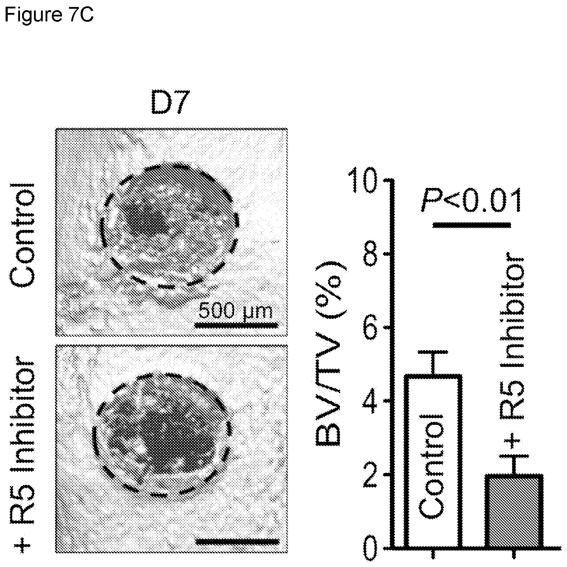

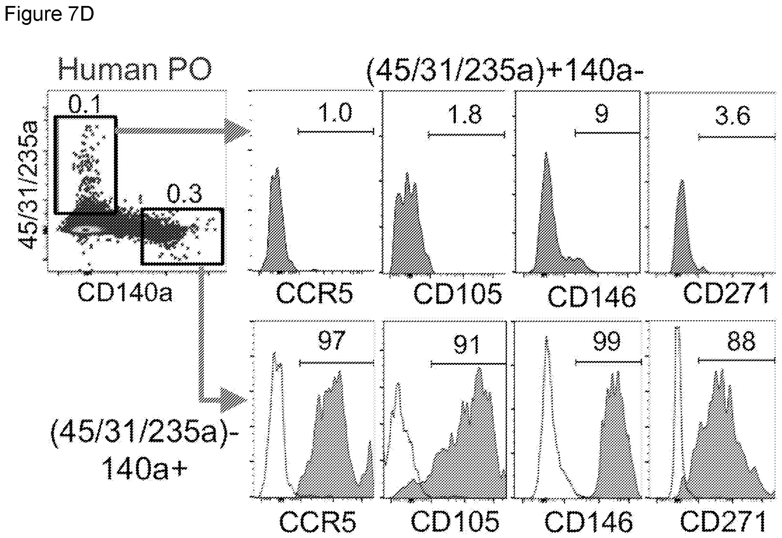

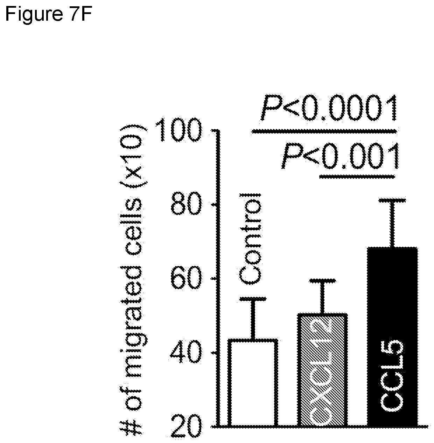

FIGS. 7A-7F show CCR5-CCL5 influences periosteal cell migration and bone healing. **FIG. 7A****.** The external callus volume (left), BV/TV (right), and mCT images of proximal tibial bone injuries in WTor Ccr5^{-/-} mice at 10 days post injury were used to assess bone healing. **FIG. 7B****.** Sequential *in vivo* imaging of Mx1/Tomato/αSMA-GFP mouse (3-month-old) calvarial injuries treated with a CCR5 inhibitor (+R5 Inhibitor; 10 mL at 2.5 mg/mL Maraviroc in Matrigel) or gel control (10 mL of Matrigel) for 10 days post injury. **FIG. 7C****.** Representative mCT images on day 7 post injury **(****FIG. 7C****,** left) and BV/TV analysis **(****FIG. 7C****,** right graph) were used for the quantification of bone healing. **FIG. 7D****.** Primary cells isolated from human periosteal tissue (Human PO) were analyzed for expression of CCR5 and indicated SSC markers. Dotted line, isotype controls. **7 FIG. E.** CCL5 induces human periosteal cell migration. The effects of CCL5 (20 ng/mL) or TNFa (20 ng/mL) on the migration of human periosteal cells (10,000/well) were determined by *in vitro* scratch assay and counting the migrating cells within 6 hours. **FIG. 7F****.** A transwell migration assay (8-µm pore) was used to determine the effects of CCL5 (5 ng/mL) or Cxcl12 (5 ng/mL) on human periosteal migration (P1, 15,000/well) migration for 24 h.

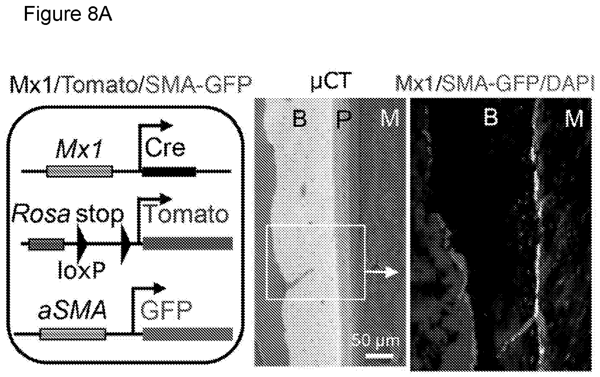

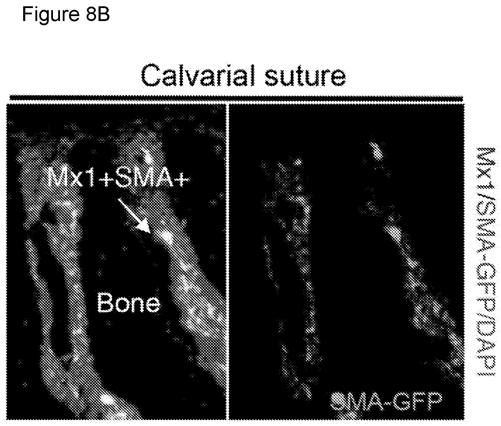

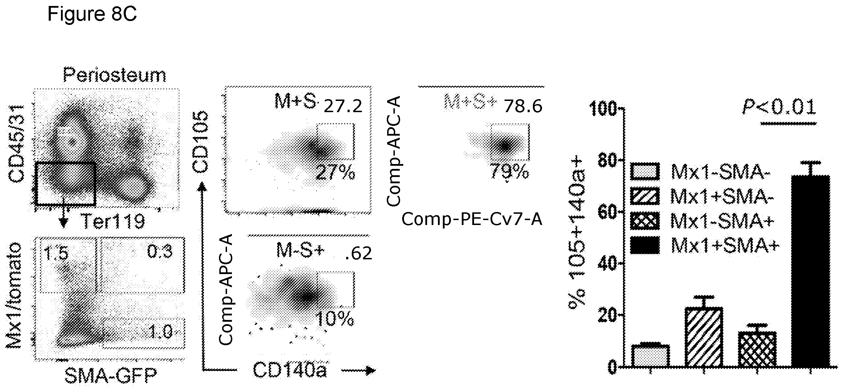

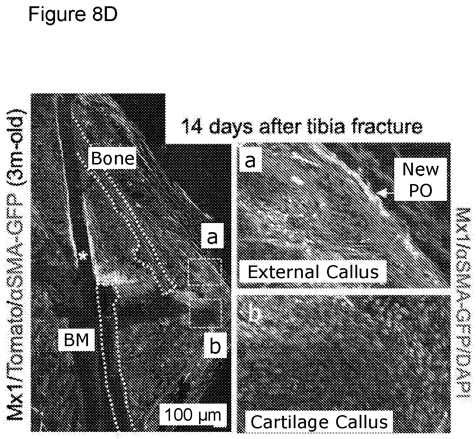

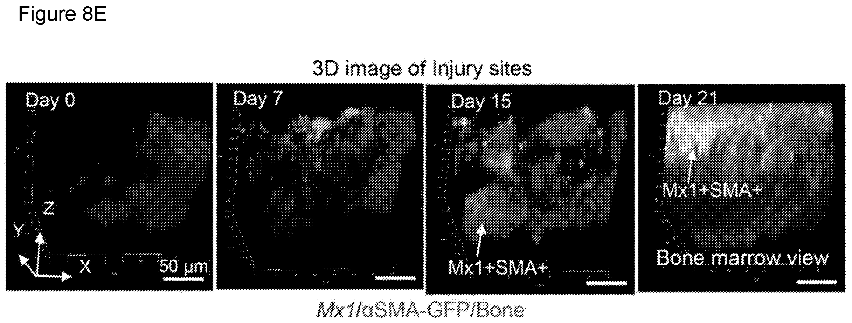

FIGS. 8A-8E *show Mx1⁺αSMA⁺* P-SSCs are the source of new osteoblasts in fracture healing and remain in the periosteum after injury, related to FIGS. 1A & 1B. The periosteal *Mx1*⁺*αSMA*⁺ population in the tibia **(****FIG. 8A****)** and calvaria suture **(****FIG. 8B****)** of Mx1/Tomato/SMA-GFP dual reporter mice was analyzed by immunofluorescence staining. Arrows indicate *Mx1*⁺*αSMA*⁺ cells (red and green) in the periosteum **(****FIG. 8A****)** and suture **(****FIG. 8B****),** *Mx1⁺αSMA⁻* osteoblasts (Mx1⁺ Obs, red) in the endosteum, and *αSMA⁺* cells (SMA+, green) in muscle. M: muscle; B: bone; BM: bone marrow. **FIG. 8C****.** High expression of CD105 and CD140a SSPC markers in the *Mx1*⁺*αSMA*⁺ fraction in the periosteum. Graph shows percentages of the CD105⁺CD140a⁺ SSC population within *Mx1⁺αSMA*⁺, *Mx1⁻αSMA*⁺, or *Mx1*⁺*αSMA⁻* cells from the periosteum determined by flow cytometry (n=6). **FIG. 8D****.** Fourteen days after transverse tibial fracture, robust contribution of the *Mx1*⁺ (Tomato⁺) periosteal cells to the external callus (box a), repopulation of Mx1⁺αSMA⁺(Tomato ⁺GFP⁺) cells in the new periosteum (white arrow, New PO), and their contribution to cartilaginous callus (box b) were assessed by immunostaining with anti-GFP antibody. Intermedullary pin insertion (*), fractured bone (dot lines) and BM are indicated (tiled image, n=5). **FIG. 8E****.** 3-D reconstruction of *in vivo* z-stack images of injury shown in FIG. 1F. *Mx1*⁺ (tomato) and *αSMA⁺* (GFP) cells newly appear at the injury sight by day 7. By day 21 the injury is filled with more differentiated *Mxl⁺αSMA⁻*cells while *Mx1*⁺*αSMA*⁺ P-SSCs mainly reside in the outer surface of the injury.

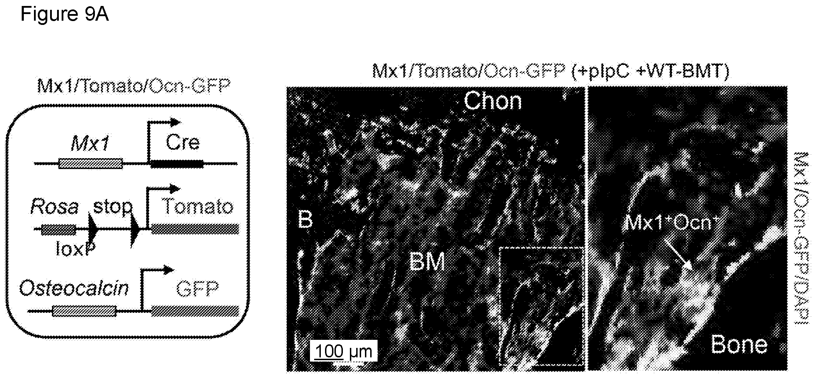

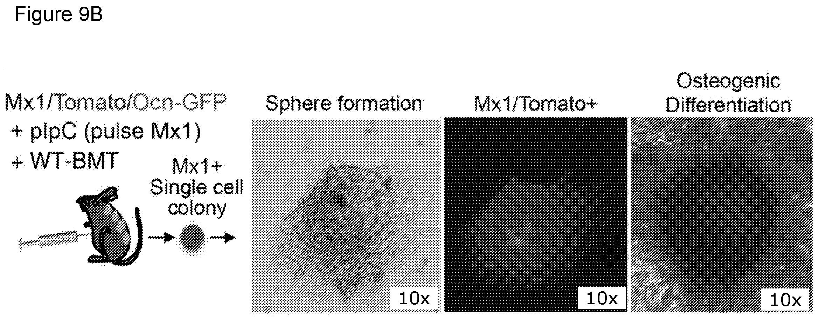

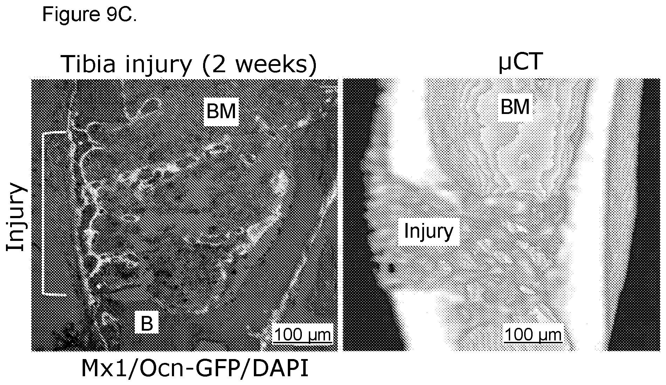

FIGS. 9A-9C show *Mx1*⁺ periosteal cells are clonogenic progenitors responsible for callus-forming cells *in vivo,* related to FIG. 2. **FIG. 9A****.** Schematic representation of *Mxl-Cre; Rosa26-Tomato; osteocalcin-GFP* mice (left) and a tibia section of Mx1/tomato/Ocn-GFP mice showing distinct metaphyseal *Mx1*⁺ progenitors (red) and their differentiated mature osteoblasts (Mx1⁺Ocn⁺, red and green) present in bone marrow 4 weeks after pIpC treatment (+pIpC), followed by lethal irradiation and wild-type bone marrow transplantation (+WT-BMT). **FIG. 9B****.** After induction of *Mxl* with pIpC and WT-BMT, single cell cultures of FACS-sorted *Mxl*⁺ periosteal cells displayed colony formation (Sphere shape) and their osteogenic differentiation potential (Alizalin staining) after 2 weeks of differentiation medium culture. **FIG. 9C****.** Robust contribution of *Mx1*⁺ periosteal progenitors (red) to the majority of new periosteal cells and callus-forming osteoblasts (red and green), without detectable responses of *Mx1*⁺ bone marrow cells (tomato) 2 weeks after proximal tibia injury.

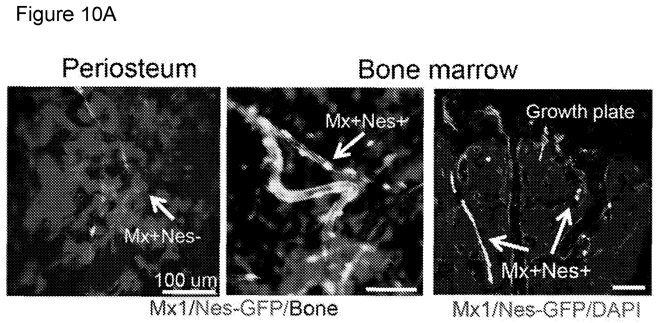

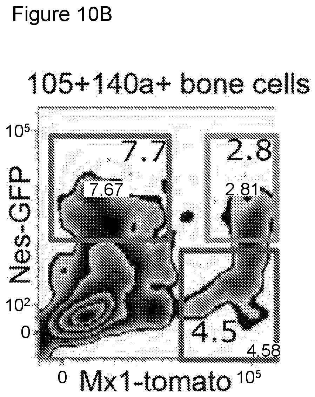

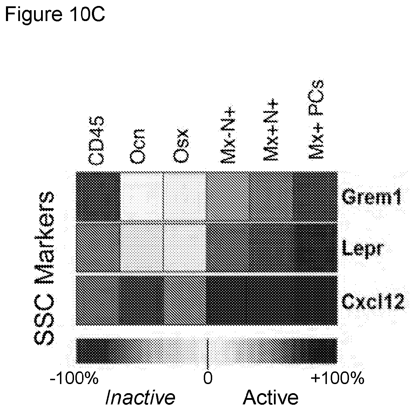

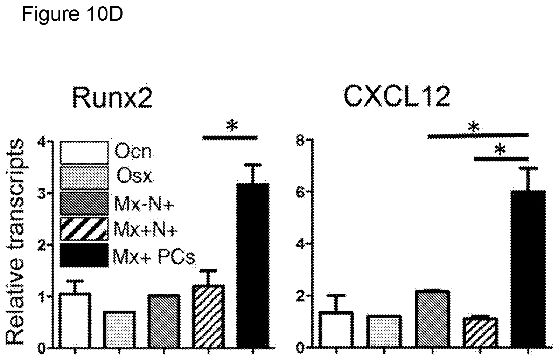

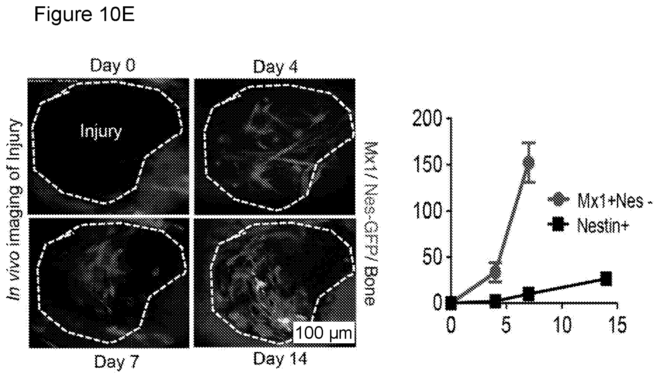

FIGS. 10A-10E show *Mxl*⁺ periosteal cells distinct from *Nestin⁺* BM-SSCs supply new osteoblasts in fracture healing, related to FIG. 3. **FIG. 10A****.** Representative *in vivo* image of the periosteum (left) and bone marrow perivascular cells (middle) and an immunofluorescence image of tibia section (right) from Mx1/Tomato/Nestin-GFP mice. **FIG. 10B****.** The percentages of *Mxl⁺Nestin⁺*, *Nestin⁺,* or *Mxl⁺* skeletal progenitors in bone were determined by flow cytometry. **FIG. 10C****.** Heatmaps representing the relative gene expression level of SSC markers compared with ~10,000 public microarray datasets (global-scale meta-analysis with Gene Expression Commons). **FIG. 10D****.** The relative expression level of SSC markers (Runx2 and Cxcl12) in the indicated cells (n=5 per group). CD45⁺ hematopoietic cells (CD45), GFP⁺ cells from *Osteocalcin-GFP* mice (Ocn), GFP⁺ cells from *Osterix-GFP* mice (Osx), and *Mx1Nestin⁺* (Mx-N+) and *Mxl⁺Nestin⁺* (Mx+N+) cells within the CD105⁺CD140a⁺ population from Mx1/Tomato/Nestin-GFP mice and *Mxl⁺Ocn⁻* periosteal cells (Mx1+ PCs) within the CD105⁺CD140a⁺ population from Mx1/Tomato/Ocn-GFP mice were used. * *P*<0.05. **FIG. 10E****.** *Mx1*⁺ SSCs (Red), but not *Nestin*-GFP⁺ cells (Green), supply the majority of osteoblasts in fracture healing. *Mx1*⁺ (Tomato) and *Nestin*-GFP⁺ cells near the injury site on calvaria of Mx1/Tomato/Nestin-GFP mice were imaged at the indicated times after injury.

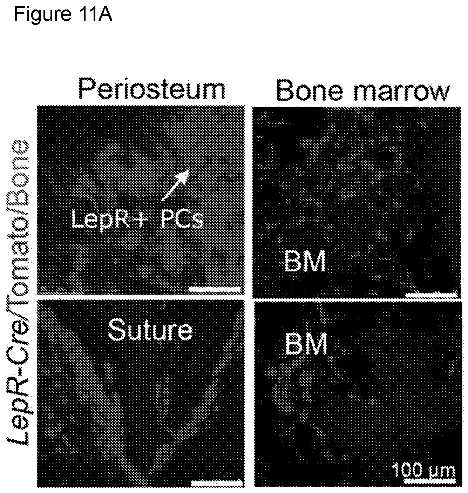

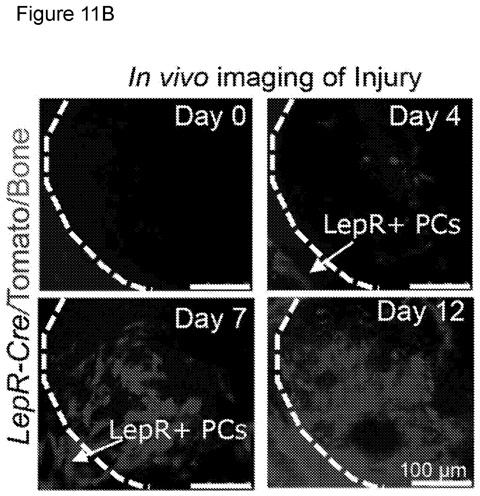

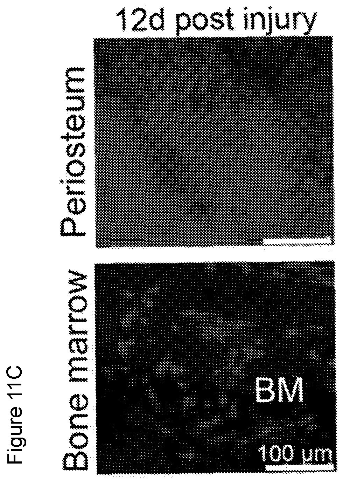

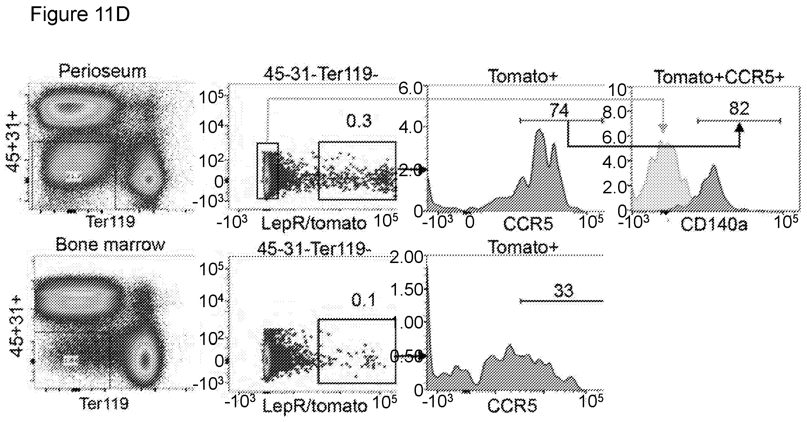

FIGS. 11A-11D show *LepR⁺* periosteal cells are distinct from *LepR⁺* bone marrow cells and supply new osteoblasts in fracture healing, related to FIG. 4. **FIG. 11A****.** *In vivo* imaging of LepR-Cre/Tomato reporter mice. *LepR⁺* (tomato) cells are present in the periosteum, suture, and bone marrow (BM) of the calvaria. **FIG. 11B****.** *In vivo* imaging of *LepR⁺* (tomato) cells in response to a calvaria injury on days 0, 4, 7, and 12 post-injury. Arrows indicate migrating and proliferating *LepR⁺* periosteal cells near the injury site. **FIG. 11C****.** *LepR⁺* cell proliferation occurs on the periosteum in response to injury, but not within the bone marrow. **FIG. 11D****.** CCR5 and CD140a expression of *LepR⁺* cells isolated from the periosteum or bone marrow was analyzed by flow cytometry.

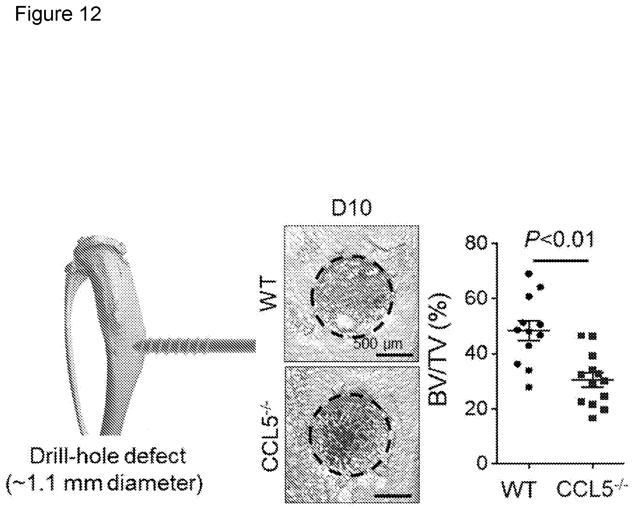

FIG. 12 shows an example of a 1.1 mm tibia drill-hole defect was created (left) and injury repair was assessed 10 days later. Representative µCT images of proximal tibia injuries (Middle) of age and sex-matched WT or 2-4-month-old CCL5^{-/-} mice ten days post-injury. Bone volume/total volume (BV/TV) was used to quantify bone healing (right).

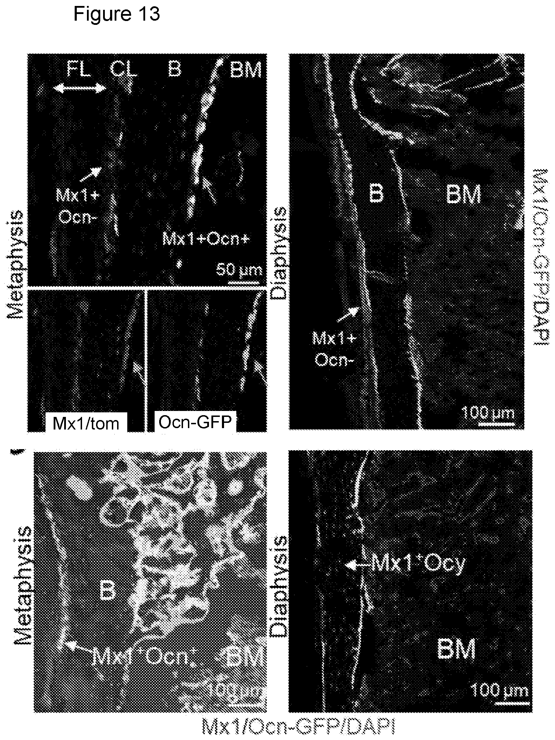

FIG. 13 shows tibia sections of Mx1/Tomato/Ocn-GFP mice two weeks after pIpC treatment demonstrating that most periosteal Mx1⁺ cells are undifferentiated (Mx1⁺Ocn⁻) and reside in the inner cambium layer (CL), rather than the fibrous layer (FL). Differentiated osteoblasts (Mx1⁺Ocn⁺, red and green) in the endosteal surface are indicated by green arrows (tiled image, n=5). C. Increase in Mx1□ progenitor contribution to the periosteal osteoblasts and osteocytes (right bottom) over time. 16 weeks after pIpC induction, newly differentiated osteoblasts (Mx1⁺Ocn⁺: red and green) and osteocytes (Mx1⁺ Ocy) from Mx1⁺ periosteal progenitor cells in the periosteum and endosteum of Mx1/Tomato/Ocn-GFP mice (~6-month old, 16 weeks post-pIpC) were analyzed by anti-GFP immunofluorescent staining (n=3).

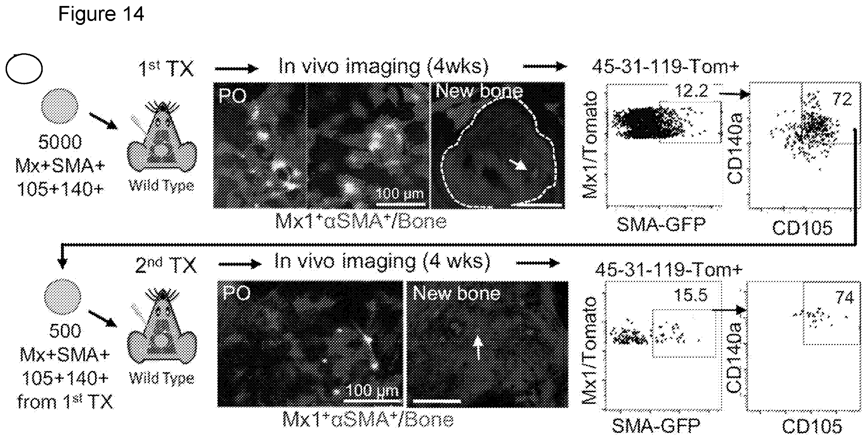

FIG. 14 shows serial transplantation of Mx1⁺αSMA⁺ P-SSCs. Four weeks after transplantation of sorted Mx1⁺αSMA⁺ P-SSCs onto calvarial injury sites of C57BL/6 mice (~5,000 cells/mouse), repopulating Mx1⁺αSMA⁺ P-SSCs in the periosteum (PO, top left, tiled images, Tomato⁺GFP⁺) and their osteogenic differentiation (topmiddle, white arrow) in newly generated bones (blue, middle dotted line) were analyzed by *in vivo* imaging. After re-sorting and transplantation of Mx1⁺αSMA⁺ P-SSCs (top right) into secondary injuries of C57BL/6 mice (~500 cells/mouse), their periosteal repopulation (PO, bottom left), osteogenic differentiation (bottom middle, white arrow) in new bones (blue), and SSC marker expression (CD105 and CD140a, bottom right) were analyzed by *in vivo* imaging and flow cytometry. As controls, transplantation of CD105⁺CD140a⁺Mx1⁺αSMA⁻ or CD140a⁺Mx1⁻αSMA⁺ periosteal cells was also performed (Figure S3). n = 5 mice per group.

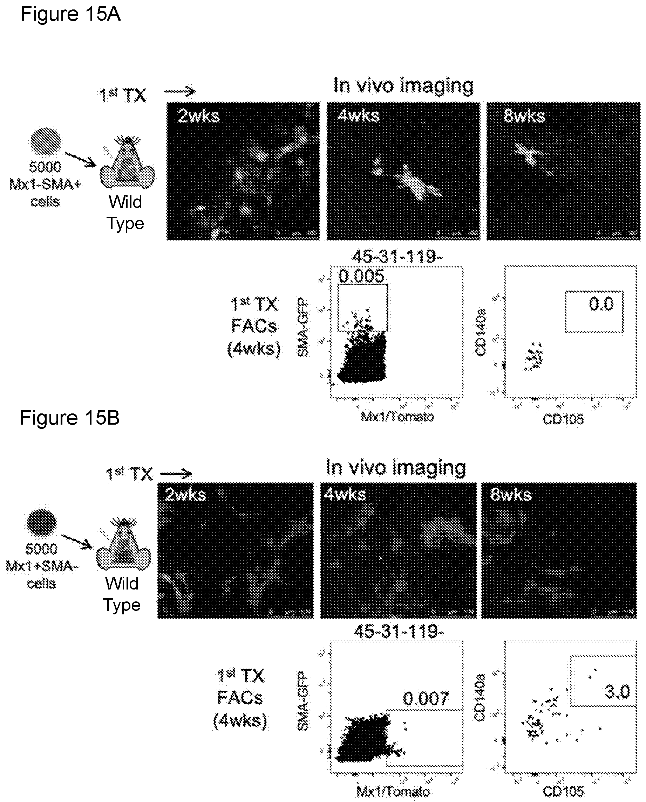

FIGS. 15A-15B show periosteal cell transplantation related to Figure 3C. Mx1/Tomato/αSMA-GFP dual reporter mice were treated with 5 doses of pIpC every other day to induce Mx1 labeling (red) at 4 weeks of age, followed by lethal irradiation and WT-BMT. Periosteal cells from 10-12-week old Mx1/Tomato/αSMA-GFP mice were FACs-sorted for CD45-CD31-Ter119-CD105⁺CD140⁺Mx1⁺αSMA-(Mx1⁺αSMA-) and CD45-CD31-Ter119-CD105⁺CD140⁺Mx1-αSMA⁺ (Mx1-αSMA⁺) cell populations. Approximately 5,000 Mx1-αSMA⁺ **(****FIG. 15A****)** or Mx1⁺αSMA- **(****FIG. 15B****)** cells were transplanted onto calvarial injury sites of wild-type C57BL/6 mice. Transplanted cells were then tracked using intravital microscopy 2, 4, and 8 weeks post transplantation. Four weeks after transplantation, transplanted cells were FACs-analyzed for SSC markers (CD105 and CD140a). Data represent five independent transplants per group with comparable results.

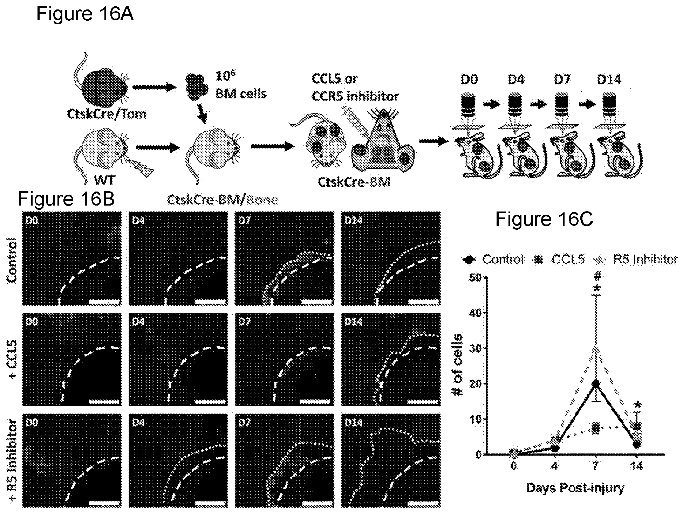

FIGS. 16A-16C show *CathepsinK (Ctsk)⁺* osteoclast fracture healing response with CCL5 treatment or CCR5 inhibition. **FIG. 16A****.** Wildtype mice were lethally irradiated and transplanted with bone marrow from *CtskCre*/*Tom* mice (CtskCre-BM) at 7 weeks of age. Calvarial injuries were induced in CtskCre-BM transplanted mice 14 weeks after transplantation, which allowed adequate time for bone marrow repopulation. Mice received one of 3 treatments at the injury sight: 1) control (2 uL Matrigel) Day 0, 2, and 4; 2) CCL5 (2 uL, 10ng/uL in Matrigel) Day 0, 2, and 4; or 3) a CCR5 inhibitor (10 uL, 4.9 mM Maraviroc in Matrigel) on Day 0, 2, 4, 7, and 10 post-injury. *Ctsk* + osteoclasts were tracked by *in vivo* imaging at the indicated time points. **FIG. 16B****.** Intravital microscopy was performed on the day of injury as well as day 4, 7, and 10 post-injury. Bone collagen (blue), *Ctsk* ⁺ osteoclasts (red), original injury (white dotted line), resorption expanding the injury (yellow dotted line). Scale bars are 100 µm. **FIG. 16C****.** The average number of *Ctsk* ⁺ osteoclasts at the injury sight were plotted from different injuries at the indicated time points. *p < 0.05 control vs. CCL5 and ^{#}p < 0.05 CCL5 vs. CCR5 inhibitor at specified time point.

### DETAILED DESCRIPTION

### I. Examples of Definitions

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure. It is contemplated that any method or composition described herein can be implemented with respect to any other method or composition described herein.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular aspects of the disclosure, the terms "about" or "approximately" when preceding a numerical value indicates the value plus or minus a range of 15%, 10%, 5%, or 1%. With respect to biological systems or processes, the term can mean within an order of magnitude, preferably within 5-fold, and more preferably within 2-fold, of a value. Unless otherwise stated, the term 'about' means within an acceptable error range for the particular value.

As used herein, the term "administer" or "administration" refers to providing a therapeutically effective amount of a composition to an individual at a site in need. The method of administration may or may not include injection, such as with a syringe, or delivery, such as implantation of the composition at the site in need. Other delivery routes are encompassed in the disclosure and detailed elsewhere herein.

When referring to "cells", it is meant any number of cells, including a single cell, that is sufficient to induce or perform the disclosed method.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, treatment" can refer to a 10%, 20%, 30%, 40%, 50%, 60%, 70%, 80%, 90%, or 100% reduction in the severity of an established disease or the disease progression, including reduction in the severity of at least one symptom of the disease. For example, a disclosed method for reducing the immunogenicity of cells is considered to be a treatment if there is a detectable reduction in the immunogenicity of cells when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition. In specific aspects of the disclosure, treatment refers to the lessening in severity or extent of at least one symptom and may alternatively or in addition refer to a delay in the onset of at least one symptom.

### II. Methods of Enhancing Bone Formation

Particular aspects of the disclosure are directed towards methods of administration of compositions to one or more sites in need, such as a bone, in an individual. The site in need may be a bone break, bone fracture, bone degeneration, and so forth The individual may have an injury, osteoporosis, osteopetrosis, osteosclerosis, or other bone degenerative diseases. For example, the invention provides a composition comprising CCL5 for use in method of treating bone break, bone fracture, bone degeneration, or osteoporosis in an individual, the method comprising administering the composition to the individual at a site in need thereof. In some aspects, the site may be one that would benefit from prophylactic systems, methods, or compositions to delay the onset or inhibit bone degeneration partially or fully, for example. The individual may also be administered other conventional or novel methods and/or compositions to aid in the bone healing process. Such methods include, for example, surgery, placing the individual in a cast or sling, a fixation device, or a combination thereof.

In particular aspects, the methods are directed towards modulation of specific skeletal stem cells (SSCs), including periosteal SSCs (P-SSCs), in an individual. The P-SSCs may be positive for the Mx1 marker. These P-SSC may have the ability to differentiate into osteoblasts, which can aid in repairing a site in need. The methods disclosed herein may induce migration of the P-SSCs to a specific site by administration of one or more compositions at the specific site(s). In particular aspects, the recruitment of P-SSCs, including those that differentiate into osteoblasts, reduces the recruitment of cells that may be, or differentiate into, osteoclasts. In some aspects, the methods are directed towards modulation of SSCs, including osteoclast progenitors (OCPs), which may express the CathespsinK marker. OCPs may have the ability to differentiate into osteoclasts, which can reduce the healing response at the site in need.

In particular aspects of the disclosure, the method is directed to induce bone healing at a site in need comprising the administration of at least one composition, such as a cytokine and/or stem cell. The site in need may be a bone break, bone fracture, bone degeneration, osteoporosis, or other bone degenerative diseases, or a site that would benefit from prophylactic systems, methods, or compositions to delay or stop bone degeneration, for example. Bone healing may be induced by recruitment of P-SSCs to the site in need, wherein the P-SSCs contribute to bone healing, such as by differentiating into osteoblasts, for example. The cytokine administered to the site in need may be CCL5, TNFα, BMP2, Wnt, or a combination thereof. One specific combination may be CCL5 and TNFα. The stem cell administered may be a P-SSC, including an Mx1⁺αSMA⁺ P-SSC, as one example. In some cases, the stem cell is administered with the one or more cytokines. In a specific aspect, a P-SSC, such as Mx1⁺αSMA⁺ P-SSC, is administered with CCL5, TNFα, BMP2, Wnt, or a combination thereof. The P-SSC may be administered with CCL5 and/or TNFα, in some cases. The invention provides a composition comprising CCL5 for use in method of treating bone break, bone fracture, bone degeneration, or osteoporosis in an individual, the method comprising administering the composition to the individual at a site in need thereof.

In any method described herein, the one or more cytokines and/or stem cells (including P-SSCs) are both provided to an individual for the purpose of enhancing bone formation and optionally may include an additional therapy for bone formation. In such cases, the one or more cytokines, the stem cells, and/or the additional therapy are administered to the individual at the same time or in succession. In cases wherein the one or more cytokines, the stem cells, and/or the additional therapy are administered to the individual in succession, the order of delivery may be in any suitable order. In some cases, the individual is administered the one or more cytokines before, during, or after the stem cells and/or additional therapy. In some cases, the individual is administered the stem cells before, during, or after the one or more cytokines and/or additional therapy. In some cases, the individual is administered the additional therapy before, during, or after the one or more cytokines and/or stem cells.

When the individual is administered CCL5, TNFα, BMP2, Wnt, or a combination thereof, the form of the CCL5, TNFα, BMP2, Wnt, or a combination thereof may be of any kind, including in polynucleotide form, such as on a vector of any kind (viral or non-viral), or in polypeptide form. In some cases, the CCL5, TNFα, BMP2, Wnt, or a combination thereof are provided in their full form, whereas in other cases the CCL5, TNFα, BMP2, Wnt, or a combination thereof are provided in a functional fragment or derivative; in such cases, a fragment or derive is function if after delivery it is able to enhance bone formation.

### III. Methods of Inhibiting Bone Formation

In particular aspects described herein for illustrative purposes, an individual is in need of inhibiting bone formation, such as at a particular site. The inhibition of bone formation may be at the site of a bone spur (including heel spurs) or site of ectopic bone formation or bunion(s), for example. The bone spur may be caused by local inflammation, such as from degenerative arthritis (osteoarthritis) or tendinitis. The ectopic bone formation may be caused by surgery or trauma, such as to the hips and legs, for example from joint replacement (for example, from total hip arthroplasty) and/or a severe fracture of bone, such as the long bones of the lower leg. Such inhibition of bone formation may or may not include the induction of migration of cells that are osteoclasts or that differentiate into osteoclasts to a site in need of bone inhibition.

In other particular aspects described herein for illustrative purposes, the composition(s) to be administered comprises an agent that inhibits CCR5, such as maraviroc, for example. The inhibition of CCR5 may induce the migration of cells that are, or differentiate into, osteoclasts, such as OCPs, for example. The site in need in such methods may including sites of ectopic bone formation, such as in muscle or tendon tissue. Ectopic bone formation can be present at birth, be the result of genetics, or arise as a complication of certain medical conditions such as paraplegia and/or traumatic injury, as examples. The composition to be administered may comprise other materials, such as a biocompatible gel and/or scaffold, for example.

An individual in need of inhibition of bone formation may be determined to be in need of inhibition of bone formation, such as upon determination of ectopic bone formation or upon identification of a bone spur or bunion, or upon identification of a risk thereof.

### IV. Cytokines and Inhibitors

In particular aspects described herein, the methods disclosed are directed to administration of a composition at a site in need, such as a bone, in an individual. The composition, in some embodiments, comprises at least one cytokine. The cytokine may be CCL5 and/or TNFα. The cytokine may be produced by any known means of obtaining cytokines, including recombinant protein purification, mammalian expression constructs, viral or non-viral transfections, isolation from cellular or hematopoietic sources, or purchasing from a commercial vendor, for example. The cytokine may also be produced in the individual from a polynucleotide or viral gene therapy. The composition may be delivered in a cell, in some cases (MSCs or isolated skeletal stem cells).

In particularaspects of the disclosure, such as in the composition for use of the invention, the cytokine comprises the CCL5 cytokine, wherein the CCL5 cytokine is translated from the CCL5 coding sequence, including as exemplary sequences, the sequences encoded by GenBank^{®} Accession No NM_001278736.1 (SEQ ID NO:1) or NM_002985.2 (SEQ ID NO:2), or any homologous sequence from a human or non-human species that produces the same function as the exemplary sequence provided. In cases wherein the CCL5 cytokine is employed as a polypeptide, examples of the polypeptide include GenBank Accession No. NP_001265665.1 (SEQ ID NO:3) or NP_002976.2 (SEQ ID NO:4).

In particular aspects of the disclosure, the cytokine comprises the TNFα cytokine, wherein the TNFα cytokine is translated from the TNFα coding sequence, including as an exemplary sequence, the sequence encoded by GenBank^{®} Accession No NM_000594.3 (SEQ ID NO:5), or any homologous sequence from a human or non-human species that produces the same function as the exemplary sequence provided. In cases wherein the TNFα cytokine is employed as a polypeptide, an example of the polypeptide encoded by GenBank Accession No. NM_000594.3 (SEQ ID NO:6).

In particular aspects of the disclosure, the cytokine comprises the BMP2 cytokine, wherein the BMP2 cytokine is translated from the BMP2 coding sequence, including as an exemplary sequence, the sequence encoded by GenBank^{®} Accession No NM_001200.3 (SEQ ID NO:7), or any homologous sequence from a human or non-human species that produces the same function as the exemplary sequence provided. In cases wherein the BMP2 cytokine is employed as a polypeptide, an example of the polypeptide includes GenBank Accession No. NP_001191.1 (SEQ ID NO:8).

In particular aspects of the disclosure, the cytokine comprises the Wnt cytokine, wherein the Wnt cytokine is translated from the Wnt coding sequence, including as an exemplary sequence, (Wnt3a in mouse bone injury is reported to induce bone healing and regeneration; Science Translational Medicine 28 Apr 2010:Vol. 2, Issue 29, pp. 29ra30), or any homologous sequence from a human or non-human species that produces the same function as the exemplary sequence provided.

Homologous sequences may be sequences that have silent mutations, wherein the homologous sequence produces the same amino acid sequence. Homologous sequences may also, in a non-limiting example, be sequences that have mutations that code for amino acids that are functionally or practically equivalent to the amino acid coded in the exemplary sequence. Homologous sequences may also have insertions or deletions relative to the exemplary sequence, wherein the insertions or deletions do not alter the inherent function of the translated cytokine. Homologous sequences may also be from a non-human species that encode for a protein or polypeptide that is functionally equivalent to CCL5, TNFα, BMP2, or Wnt. Methods to produce cytokines that utilize non-natural amino acids, such as to enhance the function of the cytokine or improve the stability of the cytokine, may also be used. In certain aspects of the disclosure, the cytokine may comprise a biologically active portion or fragment of any of the provided full length cytokines that may be administered to the individual. The fragment or portion may comprise approximately 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the amino acids of the full length cytokine. Particular aspects disclosed herein provide the use of at least one cytokine, protein, or polypeptide, or at least one polynucleotide encoding a cytokine, protein, or polypeptide, that functions equivalently to any one of the cytokines selected from the group consisting of CCL5, TNFα, BMP2, Wnt, or a combination thereof.

In cases where a CCL5, TNFα, BMP2, or Wnt polynucleotide is utilized in methods of the disclosure, the polynucleotide may be at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO:1, SEQ ID NO:2, SEQ ID NO:5, or SEQ ID NO:7, respectively. In aspects of the disclosure where a CCL5, TNFα, BMP2, or Wnt polypeptide is utilized in methods of the disclosure, the polypeptide may be at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO:3, SEQ ID NO:4, the polypeptide encoded by SEQ ID NO:6, or SEQ ID NO:8, respectively. In certain aspects of the disclosure, the polypeptide may be a fragment of a polypeptide of SEQ ID NO:3, SEQ ID NO:4, the polypeptide encoded by SEQ ID NO:6, or SEQ ID NO:8, respectively; the fragment may be at least 25, 50, 75, 100, 125, 150, 200, 225, 250, 275, 300, 325, 350, 375, 400, 425, 450, 475, 500, and so forth.

In particular aspects of the disclosure, the composition administered to the site in need is an agent that inhibits CCR5, such as maraviroc, aplaviroc, vicriviroc, or related agents. The composition may also include an agent that inhibits the function of CCL5, TNFα, BMP2, or Wnt. The agents may be antibodies, including neutralizing antibodies, humanized antibodies, scFvs, or a combination thereof. Examples of antibodies that target CCR5 include those described by Olson and Jacobson (Curr Opin HIV AIDS, 2010). Examples of antibodies that target CCL5 include those described by Aldinucci et al (International Journal of Cancer, 2007) and Glass et al (The Journal of Immunology, 2004),. Examples of antibodies that target TNFα include those described by Chatterjea et al (F1000Res, 2013) and Williams et al (PNAS, 1992). Examples of antibodies that target BMP2 include those described by Su et al (JBC, 2007).

One skilled in the art can determine the therapeutically effective amount of composition to administer to a site in need in an individual. Determination of efficacy can be determined by monitoring the site that has been administered the composition, such as by X-ray imaging or a physical examination. In particular aspects of the disclosure, the amount of composition used may be approximately at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 ng either in total or per kilogram of the individual to be administered. In specific aspects of the disclosure, the amount of composition used may be approximately at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 mg either in total or per kilogram of the individual to be administered. In some aspects of the disclosure, the amount of composition used may be approximately at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 mM of agent to be administered to the individual. In some aspects of the disclosure, the administered concentration is or is about 10, 20, 25, 30, 40, 50, 60, 70, 75, 80, 90, 100, 110, 120, 125, 130, 140, or 150 ng/ml. Ranges may be or may be about 10-150, 10-140, 10-130, 10-125, 10-120, 10-110, 10-100, 10-90, 10-80, 10-75, 10-70, 10-60, 10-50, 10-40, 10-30, 10-20, 20-150, 20-140, 20-130, 20-130, 20-125, 20-120, 20-110, 20-100, 20-90, 20-80, 20-70, 20-60, 20-50, 20-40, 20-30, 30-150, 30-140, 30-130, 30-125, 30-120, 30-110, 30-100, 30-90, 30-80, 30-75, 30-70, 30-60, 30-50, 30-40, 40-150, 40-140-, 40-130, 40-125, 40-120, 40-110, 40-100, 40-90, 40-80, 40-70, 40-60, 40-50, 50-150, 50-140, 50-130, 50-125, 50-120, 50-110, 50-100, 50-90, 50-80, 50-70, 50-60, 60-150, 60-140, 60-130, 60-125, 60-120, 60-110, 60-100, 60-90, 60-80, 60-75, 60-70, 70-150, 70-140, 70-130, 70-125, 70-120, 70-110, 70-100, 70-90, 70-80, 70-75, 80-150, 80-140, 80-130, 80-120, 80-110, 80-100, 80-90, 90-150, 90-140, 90-130, 90-125, 90-120, 90-110, 90-100, 100-150, 100-140, 100-130, 100-125, 100-120, 100-110, 110-150, 110-140, 110-130, 110-125, 110-120, 120-150, 120-140, 120-130, 120-125, 130-150, 130-140, or 140-150 ng/ml.

The number of administrations may be determined by one skilled in the art. In some aspects of the disclosure, a single administration may be used, while in further aspects of the disclosure, multiple administrations are given to the individual.

### IV. Biocompatible gel and/or scaffold

In particular aspects of the disclosure, the composition administered to the site in need may comprise a biocompatible gel and/or scaffold. Any biocompatible gel and/or scaffold may be used, wherein the gel and/or scaffold may enhance the efficacy of the cytokine or agent described above. The gel and/or scaffold may be comprised of matrigel, hydrogel, periosteum, hydroxyapatite, tricalcium phosphate, polystyrene, poly-l-lactic acid, polyglycolic acid, poly-dl-lactic-co-glycolic acid, collagen, proteoglycans, alginate-based substrates, chitosan, collagen-GAG, extracellular matrix, or a combination thereof. The gel and/or scaffold may be produced using any method known in the art. Gels and/or scaffolds that require being produced from an *in vivo* source, such as periosteum, may be from a source autologous, allogeneic, syngeneic, xenogeneic, or a combination thereof to the individual to be administered the composition at the site in need.

In particular aspects of the disclosure, the cytokine(s) and/or agent(s) is/are combined with the biocompatible gel and/or scaffold in order to produce a new composition. The new composition may be used in the methods of the present disclosure. For example, CCL5 may be combined with matrigel to form a new composition that may be administered to a site in need, such as a bone break, in order to recruit P-SSCs to differentiate into osteoblasts and induce bone healing, while reducing the recruitment of OCPs, and thus reduce osteoclasts recruitment to the site in need.

### V. Pharmaceutical Preparations

Pharmaceutical compositions of the present disclosure comprise an effective amount of one or more cytokines, CCR5 inhibitors, TNFα inhibitors, stem cells or additional agents dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of an pharmaceutical composition that contains at least one cytokine, CCR5 inhibitor, TNFα inhibitor, or additional active ingredient will be known to those of skill in the art in light of the present disclosure, as exemplified by Remington: The Science and Practice of Pharmacy, 21st Ed. Lippincott Williams and Wilkins, 2005. Moreover, for animal (*e.g.*, human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the pharmaceutical compositions is contemplated.

The cytokine or CCR5 inhibitor or TNFα inhibitor may comprise different types of carriers depending on whether it is to be administered in solid, liquid or aerosol form, and whether it need to be sterile for such routes of administration as injection. The composition of the present disclosure can be administered intravenously, intradermally, transdermally, intrathecally, intraarterially, intraperitoneally, intranasally, intravaginally, intrarectally, topically, intramuscularly, subcutaneously, mucosally, orally, topically, locally, inhalation (*e.g*., aerosol inhalation), injection, infusion, continuous infusion, localized perfusion bathing target cells directly, via a catheter, via a lavage, in cremes, in lipid compositions (*e.g.*, liposomes), or by other method or any combination of the forgoing as would be known to one of ordinary skill in the art (see, for example, Remington's Pharmaceutical Sciences, 18th Ed. Mack Printing Company, 1990).

The cytokine or CCR5 inhibitor or TNFα inhibitor may be formulated into a composition in a free base, neutral or salt form. Pharmaceutically acceptable salts include the acid addition salts, *e.g*., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Upon formulation, solutions will be administered in a manner compatible with the dosage formulation and in such amount as is therapeutically effective. The formulations are easily administered in a variety of dosage forms such as formulated for parenteral administrations such as injectable solutions, or aerosols for delivery to the lungs, or formulated for alimentary administrations such as drug release capsules and the like.

Further in accordance with the present disclosure, the composition of the present disclosure suitable for administration is provided in a pharmaceutically acceptable carrier with or without an inert diluent. The carrier should be assimilable and includes liquid, semi-solid, *i.e.,* pastes, or solid carriers. Except insofar as any conventional media, agent, diluent or carrier is detrimental to the recipient or to the therapeutic effectiveness of a composition contained therein, its use in administrable composition for use in practicing the methods of the present disclosure is appropriate. Examples of carriers or diluents include fats, oils, water, saline solutions, lipids, liposomes, resins, binders, fillers and the like, or combinations thereof. The composition may also comprise various antioxidants to retard oxidation of one or more component. Additionally, the prevention of the action of microorganisms can be brought about by preservatives such as various antibacterial and antifungal agents, including but not limited to parabens (*e.g*., methylparabens, propylparabens), chlorobutanol, phenol, sorbic acid, thimerosal or combinations thereof.

In accordance with the present disclosure, the composition is combined with the carrier in any convenient and practical manner, *i.e.,* by solution, suspension, emulsification, admixture, encapsulation, absorption and the like. Such procedures are routine for those skilled in the art.

In a specific aspect of the present disclosure, the composition is combined or mixed thoroughly with a semi-solid or solid carrier. The mixing can be carried out in any convenient manner such as grinding. Stabilizing agents can be also added in the mixing process in order to protect the composition from loss of therapeutic activity, *i.e*., denaturation in the stomach. Examples of stabilizers for use in the composition include buffers, amino acids such as glycine and lysine, carbohydrates such as dextrose, mannose, galactose, fructose, lactose, sucrose, maltose, sorbitol, mannitol, *etc.*

In further aspects, the present disclosure may concern the use of a pharmaceutical lipid vehicle compositions that include at least one cytokine or CCR5 inhibitor or TNFα inhibitor, one or more lipids, and an aqueous solvent. As used herein, the term "lipid" will be defined to include any of a broad range of substances that is characteristically insoluble in water and extractable with an organic solvent. This broad class of compounds are well known to those of skill in the art, and as the term "lipid" is used herein, it is not limited to any particular structure. Examples include compounds which contain long-chain aliphatic hydrocarbons and their derivatives. A lipid may be naturally occurring or synthetic (*i.e.,* designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether and ester-linked fatty acids and polymerizable lipids, and combinations thereof. Of course, compounds other than those specifically described herein that are understood by one of skill in the art as lipids are also encompassed by the compositions and methods of the present disclosure.

One of ordinary skill in the art would be familiar with the range of techniques that can be employed for dispersing a composition in a lipid vehicle. For example, the cytokine or CCR5 inhibitor or TNFα inhibitor may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

The actual dosage amount of a composition of the present disclosure administered to an animal patient can be determined by physical and physiological factors such as body weight, severity of condition, the type of disease being treated, previous or concurrent therapeutic interventions, idiopathy of the patient and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In certain aspects of the disclosure, pharmaceutical compositions may comprise, for example, at least about 0.1% of an active compound. In other aspects, the active compound may comprise between about 2% to about 75% of the weight of the unit, or between about 25% to about 60%, for example, and any range derivable therein. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, *etc.,* can be administered, based on the numbers described above.

### A. Alimentary Compositions and Formulations

In preferred aspects of the present disclosure, the cytokine or CCR5 inhibitor or TNFα inhibitor are formulated to be administered *via* an alimentary route. Alimentary routes include all possible routes of administration in which the composition is in direct contact with the alimentary tract. Specifically, the pharmaceutical compositions disclosed herein may be administered orally, buccally, rectally, or sublingually. As such, these compositions may be formulated with an inert diluent or with an assimilable edible carrier, or they may be enclosed in hard- or soft- shell gelatin capsule, or they may be compressed into tablets, or they may be incorporated directly with the food of the diet.

In certain aspects of the disclosure, the active compounds may be incorporated with excipients and used in the form of ingestible tablets, buccal tables, troches, capsules, elixirs, suspensions, syrups, wafers, and the like (Mathiowitz et al., 1997; Hwang et al., 1998; U.S. Pat. Nos. 5,641,515; 5,580,579 and 5,792, 451). The tablets, troches, pills, capsules and the like may also contain the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, *etc.* When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. When the dosage form is a capsule, it may contain, in addition to materials of the above type, carriers such as a liquid carrier. Gelatin capsules, tablets, or pills may be enterically coated. Enteric coatings prevent denaturation of the composition in the stomach or upper bowel where the pH is acidic. See, *e.g.,* U.S. Pat. No. 5,629,001. Upon reaching the small intestines, the basic pH therein dissolves the coating and permits the composition to be released and absorbed by specialized cells, *e.g*., epithelial enterocytes and Peyer's patch M cells. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

For oral administration the compositions of the present disclosure may alternatively be incorporated with one or more excipients in the form of a mouthwash, dentifrice, buccal tablet, oral spray, or sublingual orally-administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively, the compositions may be fashioned into a tablet or solution form that may be placed under the tongue or otherwise dissolved in the mouth.

Additional formulations hat are suitable for other modes of alimentary administration include suppositories. Suppositories are solid dosage forms of various weights and shapes, usually medicated, for insertion into the rectum. After insertion, suppositories soften, melt or dissolve in the cavity fluids. In general, for suppositories, traditional carriers may include, for example, polyalkylene glycols, triglycerides or combinations thereof. In certain aspects of the disclosure, suppositories may be formed from mixtures containing, for example, the active ingredient in the range of about 0.5% to about 10%, and preferably about 1% to about 2%.

### B. Parenteral Compositions and Formulations

In further aspects of the disclosure, the cytokine or CCR5 inhibitor or TNFα inhibitor may be administered *via* a parenteral route. As used herein, the term "parenteral" includes routes that bypass the alimentary tract. Specifically, the pharmaceutical compositions disclosed herein may be administered for example, but not limited to intravenously, intradermally, intramuscularly, intraarterially, intrathecally, subcutaneous, or intraperitoneally (U.S. Pat. Nos. 6,7537,514, 6,613,308, 5,466,468, 5,543,158; 5,641,515; and 5,399,363).

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Patent 5,466,468). In all cases the form must be sterile and must be fluid to the extent that easy injectability exists. It must be stable under the conditions of manufacture and storage and must be preserved against the contaminating action of microorganisms, such as bacteria and fungi. The carrier can be a solvent or dispersion medium containing, for example, water, ethanol, polyol (*i.e.,* glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. For example, one dosage may be dissolved in isotonic NaCl solution and either added hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. A powdered composition is combined with a liquid carrier such as, *e.g*., water or a saline solution, with or without a stabilizing agent.

### C. Miscellaneous Pharmaceutical Compositions and Formulations

In other preferred aspects of the disclosure, the active compound, comprising a cytokine or CCR5 inhibitor or TNFα inhibitor may be formulated for administration via various miscellaneous routes, for example, topical (*i.e.,* transdermal) administration, mucosal administration (intranasal, vaginal, *etc.*) and/or inhalation.

Pharmaceutical compositions for topical administration may include the active compound formulated for a medicated application such as an ointment, paste, cream or powder. Ointments include all oleaginous, adsorption, emulsion and water-soluble based compositions for topical application, while creams and lotions are those compositions that include an emulsion base only. Topically administered medications may contain a penetration enhancer to facilitate adsorption of the active ingredients through the skin. Suitable penetration enhancers include glycerin, alcohols, alkyl methyl sulfoxides, pyrrolidones and luarocapram. Possible bases for compositions for topical application include polyethylene glycol, lanolin, cold cream and petrolatum as well as any other suitable absorption, emulsion or water-soluble ointment base. Topical preparations may also include emulsifiers, gelling agents, and antimicrobial preservatives as necessary to preserve the active ingredient and provide for a homogenous mixture. Transdermal administration may also comprise the use of a "patch". For example, the patch may supply one or more active substances at a predetermined rate and in a continuous manner over a fixed period of time.

In certain aspects of the disclosure, the pharmaceutical compositions may be delivered by eye drops, intranasal sprays, inhalation, and/or other aerosol delivery vehicles. Methods for delivering compositions directly to the lungs via nasal aerosol sprays has been described e.g., in U.S. Pat. Nos. 5,756,353 and 5,804,212. Likewise, the delivery of drugs using intranasal microparticle resins (Takenaga et al., 1998) and lysophosphatidyl-glycerol compounds (U.S. Pat. No. 5,725, 871) are also well-known in the pharmaceutical arts. Likewise, transmucosal drug delivery in the form of a polytetrafluoroetheylene support matrix is described in U.S. Pat. No. 5,780,045.

The term aerosol refers to a colloidal system of finely divided solid of liquid particles dispersed in a liquefied or pressurized gas propellant. The typical aerosol for inhalation will consist of a suspension of active ingredients in liquid propellant or a mixture of liquid propellant and a suitable solvent. Suitable propellants include hydrocarbons and hydrocarbon ethers. Suitable containers will vary according to the pressure requirements of the propellant. Administration of the aerosol will vary according to subject's age, weight and the severity and response of the symptoms.

### VI. Kits

Any of the compositions described herein may be comprised in a kit. In a non-limiting example, a cytokine or CCR5 inhibitor or TNFα inhibitor, lipid, and/or additional agent, may be comprised in a kit. The kits will thus comprise, in suitable container means, a cytokine or CCR5 inhibitor or TNFα inhibitor and a lipid, and/or an additional agent of the present disclosure.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the cytokine or CCR5 inhibitor or TNFα inhibitor, lipid, additional agent, and any other reagent containers in close confinement for commercial sale. Such containers may include injection or blow-molded plastic containers into which the desired vials are retained.

Therapeutic kits of the present disclosure are kits comprising a cytokine protein, polypeptide, peptide, inhibitor, gene, vector and/or other CCR5 effector. Such kits will generally contain, in suitable container means, a pharmaceutically acceptable formulation a cytokine protein, polypeptide, peptide, domain, inhibitor, and/or a gene and/or vector expressing any of the foregoing in a pharmaceutically acceptable formulation. The kit may have a single container means, and/or it may have distinct container means for each compound.

When the components of the kit are provided in one and/or more liquid solutions, the liquid solution is an aqueous solution, with a sterile aqueous solution being particularly preferred. The cytokine or CCR5 inhibitor or TNFα inhibitor compositions may also be formulated into a syringeable composition. In which case, the container means may itself be a syringe, pipette, and/or other such like apparatus, from which the formulation may be applied to an infected area of the body, injected into an animal, and/or even applied to and/or mixed with the other components of the kit.

However, the components of the kit may be provided as dried powder(s). When reagents and/or components are provided as a dry powder, the powder can be reconstituted by the addition of a suitable solvent. It is envisioned that the solvent may also be provided in another container means.

The container means will generally include at least one vial, test tube, flask, bottle, syringe and/or other container means, into which the cytokine protein, gene and/or inhibitory or CCR5 inhibitor formulation are placed, preferably, suitably allocated. The kits may also comprise a second container means for containing a sterile, pharmaceutically acceptable buffer and/or other diluent.

The kits of the present disclosure will also typically include a means for containing the vials in close confinement for commercial sale, such as, *e.g*., injection and/or blow-molded plastic containers into which the desired vials are retained.

Irrespective of the number and/or type of containers, the kits of the disclosure may also comprise, and/or be packaged with, an instrument for assisting with the injection/administration and/or placement of the ultimate cytokine protein and/or gene composition or CCR5 inhibitor within the body of an animal. Such an instrument may be a syringe, pipette, forceps, and/or any such medically approved delivery vehicle.

### VII. Examples

The following examples are included to demonstrate particular aspects of the disclosure. It should be appreciated by those of skill in the art that the techniques disclosed in the examples that follow represent techniques discovered to function well in the practice of the disclosure, and thus can be considered to constitute particular modes for its practice.

### EXAMPLE 1

### THE MX1 AND ASMA COMBINATION SELECTIVELY LABELS ENDOGENOUS P-SSCS.

The origin of new osteoblasts, particularly at fracture sites, has long been an important question for biologists (Schindeler et al., 2008). Recent studies revealed that a subset of periosteal cells has skeletal progenitor function and plays an important role in bone repair (van Gastel et al., 2012). However, there is no known P-SSC specific marker, making the *in vivo* origin and regulatory mechanism of these cells elusive. Studies have reported that *Mx1* labels endogenous osteogenic stem/progenitor cells that can maintain the majority (>80%) of the mature osteoblast population over time in the adult mouse (Park et al., 2012a). It was also found that these cells are present in the periosteum and supply new osteoblasts for bone healing. Recently, several models have been developed to label P-SSCs; however, one limitation of these models, like the *Mx1* model, is that both P-SSCs and multiple cell types are labeled. Therefore, to further define a model enabling selective labeling of P-SSCs, *Mx1-Cre⁺Rosa26-Tomato⁺* mice were crossed with other mesenchymal stem cell (MSC) reporter mouse lines, including *αSMA-GFP* (Grcevic et al., 2012), *Nestin-GFP* (Mendez-Ferrer et al., 2010), and *CXCL12-GFP* (Omatsu et al., 2010). Among the tested combinations, immunohistochemistry of trigenic Mxl-*Cre⁺Rosa26-Tomato⁺αSMA-GFP⁺* (Mx1/Tomato/αSMA-GFP) mice after a wild-type BM transplant showed that the *Mx1* and *αSMA-GFP* combination selectively labels a subset of periosteal cells (FIG. 1A). To test whether *Mx1*/*αSMA-GFP* double labeling (*Mx1*⁺*αSMA⁺*) can specify P-SSCs, and to define their *in vivo* location in the periosteum and in the BM, polyinosinic:polycytidylic acid (pIpC) was administered to trigenic Mx1/Tomato/αSMA-GFP mice (25 mg/kg every other day for 10 days) and transplanted wild-type BM into irradiated Mx1/Tomato/SMA-GFP mice to eliminate *Mx1*⁺ hematopoietic cells. Four to six weeks later, histological and *in vivo* image analysis of Mx1/Tomato/αSMA-GFP mouse calvaria and tibia revealed that most *Mr1⁺αSMA⁺* (Tomato⁺GFP⁺) cells reside in the periosteum and calvarial sutures (a known niche for craniofacial SSCs (FIG. 8B)) (Zhao et al., 2015), but are nearly undetectable in the BM (FIG. 1A, FIG. 1B, and FIG. 8A). Most BM-SSCs, and their colony-forming units-fibroblast (CFU-F) activity, are enriched in the non-hematopoietic (CD45⁻Ter119⁻CD31⁻) Sca1⁺CD140a⁺ (Chan et al., 2009; Morikawa et al., 2009), CD140a⁺CD51⁺ (Pinho et al., 2013) or CD105⁺CD140a⁺ fractions (Park et al., 2012a). Interestingly, fluorescence-activated cell sorting (FACS) analysis of the periosteum of Mx1/Tomato/αSMA-GFP mice showed that periosteal cells form a heterogeneous population and that ~30% of *Mx1*⁺ periosteal cells are *αSMA*-GFP positive, and approximately 80% of *Mx1*⁺*αSMA*⁺ periosteal cells express SSC immunophenotypic markers (CD105⁺CD140a⁺) with higher expression of SSC transcripts, including *Greml, LepR,* and *Cxcl12* (FIG. 1C). By contrast, only ~10% of *αSMA⁺* cells and ~27% of *Mx1*⁺ cells from the CD45⁻CD31⁻Ter119⁻fraction are CD105⁺CD140a⁺, with a ~5-fold lower expression of SSC transcripts (FIG. 1C and FIG. 8C). When cultured at the single-cell level, *Mx1*⁺*αSMA*⁺ periosteal cells can form hemisphere colonies with osteogenic, chondrogenic, and adipogenic differentiation potential *in vitro,* demonstrating that *Mx1*⁺*αSMA*⁺ periosteal cells exhibit *ex vivo* SSC characteristics and that the *Mx1* and *αSMA*-GFP combination, but not *αSMA*-GFP alone, further specifies endogenous P-SSCs *in vivo* (FIG. 1D).

Given that periosteal progenitor cells are reported to give rise to bone and cartilage in response to injury (Colnot, 2009), It was examined whether *Mx1*⁺*αSMA*⁺ periosteal cells contribute to the formation of osteoblasts during injury repair *in vivo.* To define the origin and dynamics of the osteoblasts located at injury sites, drill-hole defects (~1 mm diameter) were generated in the tibia, using a needle (20G), in Mx1/Tomato/αSMA-GFP dual reporter mice. Histology of tibia injury at day 14 revealed that *Mx1*⁺*αSMA*⁺ periosteal cells repopulate the outer layer of the callus (FIG. 1E, New PO) and supply the majority of callus-forming cells (FIG. 1E, Callus). It was previously reported that *Mx1*⁺ stem/progenitors in the BM exhibit mainly osteogenic potential, with little adipogenic and no chondrogenic potential in postnatal bone maintenance (Park et al., 2012a). Interestingly however, immunofluorescence staining of chondrocytes (anti-aggrecan) and osteoblasts (anti-osteocalcin) in the injury revealed that *Mx1*⁺*αSMA*⁺ periosteal cells contribute to 21±3% of chondrocytes in the cartilage intermediate, and over 80% of new osteoblasts within the callus, implicating that they differ from *Mx1*⁺ BM-SSCs (FIG. 1E). It was consistently found that *Mx1*⁺*αSMA*⁺ cells mainly contributed to callus formation at 14 days after a transverse tibial fracture (FIG. 8D). When *Mx1*⁺*αSMA*⁺ periosteal cells in this model were tracked during bone repair of injured calvaria *in vivo,* these cells newly appeared at the injury site and repopulated on days 7 and 15 (FIG. 1F, FIG. 8E). In addition, 21 days after injury, *in vivo* imaging with 3D reconstitution of Z-stack images showed that the endosteal space of the injury region was filled with differentiated *Mx1⁺αSMA*⁻ cells, while *Mx1*⁺*αSMA*⁺ cells exclusively resided in the outer surface and formed new periosteum at the injury site (FIG. 1F & FIG. 8D). Additionally, 21 days post-injury, *Mx1*⁺*αSMA*⁺ cells near the injured bone surface became *Mx1*⁺*αSMA*⁻ osteoblasts and osteocytes in newly formed bones (FIG. 1G). Of note, *Mx1*⁺ BM cells near the injury site showed no observable activation. These data support the idea that *Mx1*⁺*αSMA*⁺ periosteal cells include endogenous P-SSCs with a multi-lineage differentiation potential, and that the migration and proliferation of *Mx1*⁺*αSMA*⁺ P-SSCs supply the osteoblasts participating in bone healing.

### EXAMPLE 2

### MX1⁺ P-SSCS ARE THE MAJOR SOURCE OF NEW OSTEOBLASTS IN BONE HEALING IN VIVO.

It is possible that the periosteum contains *Mx1⁻* P-SSCs that can contribute to new osteoblasts at injury sites. Therefore, to distinguish between P-SSCs and mature osteoblasts in the periosteum and to improve specific cell quantitation during bone healing, trigenic *Mx1-Cre⁺Rosa26-Tomato⁺osteocalcin-GFP⁺* (Mx1/Tomato/Ocn-GFP) reporter mice were generated. In this model, when *Mx1*⁺ P-SSCs (Tomato⁺) differentiate into mature *Ocn*-GFP⁺ osteoblasts they express Tomato and GFP, while mature osteoblasts from *Mx1⁻* P-SSCs express GFP alone (Park et al., 2014). *In vivo* imaging and immunohistochemistry analysis of Mx1/Tomato/Ocn-GFP femurs and tibias revealed that the induction of *Mx1*-Cre activity can label distinct periosteal stem/progenitor cells without detectable *Ocn-*GFP expression in the metaphysis (50±4%) and diaphysis (64±6%) (FIG. 2A). In contrast to a widely mixed distribution of *Mx1*⁺ progenitors (Tomato⁺) and osteoblasts (Tomato⁺GFP⁺; ~50-60% of endosteal osteoblasts) in trabecular bones (FIG. 2B, FIG. 13 ), these *Mx1*⁺ P-SSCs exclusively exist in the cambial layer (a site reported by others to be the location of skeletal progenitors) of the calvaria and femur (FIG 2B & FIG. 2C). To determine that Mx1⁺Ocn⁻ periosteal cells contribute to normal periosteal bone growth, the percentage of Mx1¹Ocn⁺ periosteal osteoblasts and osteocytes in the tibia of Mx1/Tomato/Ocn-GFP mice at 2, 8, and 16 weeks after pIpC treatment. The number of Mx1⁺Ocn⁺ periosteal osteoblasts and osteocytes in cortical bone was markedly increased in 7-month-old mice (from ~18% at 2 weeks to ~80% at 16 weeks post pIpC) supporting that Mx1⁺Ocn⁻ periosteal cells are the major source of newly generated periosteal osteoblasts and osteocytes in postnatal bones. Immunophenotypic analysis of periosteal cells from collagenase-digested tibia collected from Mx1/Tomato/Ocn-GFP mice revealed that *Mx1*⁺ P-SSCs accounted for 48% of the non-hematopoietic CD105⁺CD140a⁺ fraction of periosteal cells and formed clonogenic mesenspheres with *in vitro* osteogenic differentiation potential (FIG. 9B). Next, to test whether P-SSCs are enriched in *Mx1*⁺ periosteal cells, *Mxl⁻* and *Mx1*⁺ cells were sorted within the SSPC (CD45⁻CD31⁻TER119⁻CD105⁺CD140a⁺) population from the periosteum. Gene expression analysis of these fractions and the control fractions revealed that the non-hematopoietic Mx1⁺CD105⁺CD140a⁺ population expressed significantly higher levels of SSC markers, including *Grem1, LepR, Cxcl12,* and *Runx2,* but no detectable levels of *Ocn* (a mature osteoblastic marker) compared with *Mxl⁻* progenitors and other populations (FIG. 2D), indicating that P-SSC properties are exclusively enriched in *Mx1*⁺ periosteal cells.

To test whether *Mr1*⁺ P-SSCs are the main source of new osteoblasts during bone repair *in vivo,* drill-hole defects (~1 mm diameter removal of bone and periosteum) were generated in the tibial diaphysis of Mx1/Tomato/Ocn-GFP mice. A drill-hole defect can potentially be a model of bone healing without compromising tissue architecture (endosteum vs periosteum) and achieve better quantification of stem cell responses (He et al., 2011). Immunohistochemistry analysis of the bone defect revealed that *Mx1*⁺ P-SSCs reconstitute the majority (~80%) of outer callus-forming cells with new periosteum (Tomato⁺), and >70% of the bone-forming osteoblasts (Tomato⁺GFP⁺) surrounding new bone bridges (FIG. 2E & 9C). Next, to investigate the specific injury response of periosteal cells, a bone defect in the periosteum was generated and the outer surface of cortical bone, without BM damage, in the tibia of Mx1/Tomato/Ocn-GFP mice. Two weeks post-injury, there was consistently observed robust callus formation with over 90% contribution of *Mx1*⁺ P-SSCs to new osteoblasts (GFP⁺) in the injury callus (FIG. 2F). Taken together, these data show that *Mx1* can label stem/progenitor cells in the periosteum and that the activation of *Mr1⁺* P-SSCs upon cortical bone injury is sufficient to induce the injury response and osteogenesis.

### EXAMPLE 3

### MX1⁺αSMA⁺ P-SSCS ARE LONG-TERM REPOPULATING PROGENITORS REQUIRED FOR BONE HEALING.

SSCs are known to be present in the BM (Worthley et al., 2015) and may contribute to bone healing if the periosteum is defective or removed. To determine if *Mxl⁺* periosteal cells are essential for bone healing, an inducible deletion model was developed by intercrossing *Mx1-Cre*/*Rosa26-Tomato* mice with *Rosa26-DTR* mice (Park et al., 2012a) and the requirement for *Mx1*⁺ periosteal cells in bone healing was tested. To restrict the conditional deletion of *Mx1*⁺ cells to the periosteum, local surface administration of diphtheria toxin (DT) (20 µL, 1 µg/mL) was used to cover an area with ~1 cm of diameter at the junction of the sagittal and coronal sutures (FIG. 3A). Treatment of the periosteum with DT for seven days prior to injury reduced the number of *Mx1*⁺ periosteal cells in more than 90%, while *Mx1*⁺ cells in the BM and in the distal periosteum remained unchanged (FIG. 3B). Under these conditions, sequential imaging of injury repair for 16 days revealed that the DT-mediated local deletion of *MX1⁺* periosteal cells completely inhibits the recruitment and proliferation of *Mx1*⁺ osteogenic cells at the injury site (FIG. 3C). It was also confirmed that local DT treatment significantly delays both calvaria and tibia bone healing by micro-computerized tomography (µCT) analysis (FIG. 3D). Since *Mxl*-labeled osteoblasts and progenitors in the BM near the injury site remained intact, and no detectable changes in their population or position occurred during injury healing, the new *Mx1*⁺ cells within the bone injury were highly likely to be derived from the periosteum. These data are consistent with *Mx1*⁺ periosteal cells being necessary for osteogenesis and bone healing.

An important characteristic of stem cells is their long-term repopulation capability after multiple rounds of injuries (Sacchetti et al., 2007). Given that a subset of *Mx1*⁺*αSMA*⁺ periosteal cells remains on the outer layer of the callus even after bone healing, the stem cell function of these cells was tested by examining the long-term repopulation ability of *Mx1*⁺*αSMA*⁺ periosteal cells and their contribution to bone repair after several rounds of injuries. Consecutive intravital imaging of the primary injury sites of Mx1/Tomato/αSMA-GFP mice at ~4 months of age (FIG. 1D), and again at ~10 months of age, revealed complete bone recovery and that *Mx1⁺αSMA⁺* periosteal cells returned to pre-injury homeostatic levels (FIG. 3E, Pre-injury). When the secondary injuries were generated near the primary injury sites and *Mx1*⁺*αSMA*⁺ periosteal cells were further traced at the secondary injury sites, they rapidly repopulated and contributed to over 80% of osteoblasts, similar to their response to the primary injury (FIG. 3E, D14). These data indicate that the *Mx1* and *αSMA*-GFP combination labels a long-term repopulating P-SSC subset that sustains itself while durably supplying the majority of new osteoblasts in bone injury over time in the adult mouse.

To better define the long-term repopulation characteristics and to exclude the possibility that Mx1⁺αSMA⁻ periosteal cells include P-SSCs and become Mx1⁺αSMA⁺ cells in bone injury, we performed serial transplantations of these cells (FIG. 14). A total of 5,000 Mx1⁺αSMA⁺CD140a⁺CD105⁺, Mx1⁺αSMA⁻, and Mx1⁻αSMA+ cells from the periosteal CD45⁻CD31⁻Ter119⁻ fraction were sorted and transplanted with Matrigel onto the calvaria injury site of a WT mouse. We reasoned that this P-SSC transplantation onto a periosteal injury site is more physiologically relevant compared to the previously described SSC transplantation into kidney capsules (Chan et al., 2015) or mammary fat pads (Debnath et al., 2018). *In vivo* imaging and FACS analysis at 4 weeks after the first round of transplantation showed that the transplanted Mx1⁺αSMA⁺ cells proliferated and differentiated into Mx1⁺ osteoblasts and osteocytes that were imbedded within the newly formed bone tissue (FIG. 14, top arrow, New bone). A subset of the engrafted cells repopulated and maintained the Mx1⁺αSMA⁺ population in the periosteum (12%-15% of all transplanted Mx1⁺ cells). The majority of the Mx1⁺αSMA⁺ cells (~72%) preserved the expression of SSPC surface markers (CD140a⁺CD105⁺) (Figure 3C, top). When these Mx1⁺αSMA⁺CD140a⁺CD105⁺ cells (~500 cells/mouse) were re-transplanted and analyzed at 4 weeks after the second transplantation, they displayed both intact repopulation of double positive cells (FIG. 14, bottom PO, ~15% of transplanted cells) with high CD140a⁺CD105⁺ expression (~74%) and their osteoblast and osteocyte differentiation (FIG. 14, bottom arrow, New bone). However, neither transplanted Mx 1⁺αSMA⁺ nor Mx1⁻αSMA⁺ cells did not show such repopulation, and there was no detectable stem cell marker expression (FIG. 15), demonstrating that the key characteristics of SSCs-*in vivo* long-term repopulation and differentiation-are highly enriched in the Mx1⁺αSMA⁺ periosteal cells.

### EXAMPLE 4

### MX1⁺ PERIOSTEAL CELLS, DISTINCT FROM NESTIN-GFP⁺ BM-SSCS, SUPPLY NEW OSTEOBLASTS IN FRACTURE HEALING

BM-SSCs were reported to localize in perivascular regions with *Nestin-GFP* expression (Mendez-Ferrer et al.). In addition, the CD31⁻*Nestin-*GFP⁺ perivascular reticular cells are *LepR-Cre* descendants and contribute to postnatal bone regeneration (Kunisaki et al., 2013). *Mx1*⁺ cells have been shown to overlap with a subset of *Nestin⁺* populations in the BM (Park et al., 2012a), and therefore researchers developed the trigenic *Mx1-Cre⁺Rosa26-Tomato⁺Nestin-GFP⁺* (Mx1/Tomato/Nestin-GFP) dual reporter mouse and tested if *Mx1* and *Nestin* double labeling can distinguish BM-SSCs from P-SSCs and their contributions to osteolineage cells in bone injury. *In vivo* imaging and FACS analysis of these mice revealed that there are two distinct cell populations (*Mx1⁺Nestin⁺*; ~27% and *⁻Nestin*⁺; ~73%) within *Nestin⁺* perivascular cells, whereas *Mx1*⁺ periosteal cells are exclusively *Nestin* negative (FIG. 10A & FIG. 10B). To determine if marker expression was different between P-SSC and BM-SSC populations, Affymetrix-based global gene expression analysis with FACS-sorted cells were performed: (1) CD105⁺CD140a⁺Mx1⁺Ocn⁻ periosteal cells from Mx1/Tomato/Ocn-GFP mouse periosteum (FIG. 2D, Mx1⁺), *(2) Mxl⁺Nestin⁺* and *(3) Mx1Nestin⁺* cells within the non-hematopoietic CD105⁺CD140a⁺ population from Mx1/Tomato/Nestin-GFP mouse bones and control cells, including (4) CD45⁺ hematopoietic cells, (5) Osteocalcin⁺ (*Ocn*⁺), and (6) Osterix⁺ (*Osx*⁺) cells from *Osteocalcin-GFP* and *Osterix-GFP* mice, respectively. Interestingly, the expression levels of SSC markers, including *Greml, LepR,* and *CXCL12,* were much higher in *Mx1*⁺*Ocn*⁻ periosteal cells compared to *Nestin⁺* BM perivascular cells and control cells. The highest expression level of *Runx2* and *CXCL12* was confirmed in *Mx1*⁺ periosteal cells by reverse transcription polymerase chain reaction RT-PCR (FIG. 10C & FIG. 10D). *Mxl⁺Nestin⁺* (Tomato⁺GFP⁺), *Mx1*⁺*Nestin*⁻ (Tomato⁺), and *Nestin⁺* (GFP⁺) cells were tracked at the bone injury site using intravital imaging and it was found *that Mx1*⁺*Nestin⁻* periosteal cells predominantly appeared at the injury site at 2-5 days (>95%) and subsequently increased in number. In marked contrast, few *Mx1*⁺*Nestin*⁺ (2-3%) or *Mxl⁻Nestin⁺* (~1%) cells were observed at the injury site throughout the repair process (FIG. 10E). Taken together, these results demonstrate that *Mxl*⁺ subsets in the periosteum have SSC properties, respond to injury, and supply new osteoblasts.

### EXAMPLE 5

### MX1⁺ASMA⁺ P-SSCS HAVE A UNIQUE MIGRATORY MECHANISM REGULATED BY CCR5 IN VIVO.

Skeletal progenitors have been proposed to have migratory or circulatory potential. However, there is no *in vivo* evidence for the endogenous migratory mechanism of P-SSCs. In particular, how P-SSCs are signaled to respond to injury and how they migrate and form fracture-repairing osteoblasts is essentially unknown. To test whether P-SSCs migrate toward the injury site *in vivo,* continuous Z-stack imaging of individual *Mx1*⁺*αSMA*⁺ P-SSCs was performed and sequentially scanning was performed on the detailed structures of the periosteum, bone matrix, and BM near the injury sites immediately (0), 24, and 48 hours after injury (FIG. 4A). Multiple *Mx1*⁺*αSMA*⁺ P-SSCs appeared to move toward the injury site (FIG. 4A, arrows), and, which was confirmed by Z-stack imaging. However, in agreement with FIG. 3D, the endosteal and vessel-associated *Mx1*⁺ cells were not observed to shift their position, indicating the presence of a unique migratory mechanism of *Mxl⁺αSMA*⁺ P-SSCs and suture cells.

Many growth factors and cytokines are known to enhance the migration and proliferation of *in vitro*-expanded MSCs (Einhorn and Gerstenfeld, 2015; Schindeler et al., 2008). However, there is no direct evidence of the effect of these molecules on the *in vivo* migration potential of endogenous SSCs. molecules involved in P-SSC migration were examined by Affymetrix-based global gene expression analysis of *Mxl⁺Ocn⁻* periosteal cells compared to more differentiated cells and *Nestin⁺* BM cells (FIG 10C). Interestingly, It was found that two specific CC chemokine receptors, *CCR3* and *CCRS,* are highly expressed in *Mxl⁺* periosteal cells (FIG. 4B). It was also found that there was increased expression of genes involved in MSC migration and proliferation, including *Integrin α3, Icaml,* and *FGFS.* Consistently, Gene Set Enrichment Analysis (GSEA) showed a significant upregulation of the filopodia-associated migratory gene set (Gene Set: MILI_PSEUDOPODIA_CHEMOTAXIS _DN) in *Mx1*⁺ periosteal cells compared to more mature osteoblasts (FIG. 4C). Subsequent FACS and real-time PCR analysis determined that almost all *Mx1*⁺*αSMA*⁺ P-SSCs express CCR5 (92%) and CCR3 (90%) on the cell surface at levels comparable to CD45⁺ hematopoietic cells, while more differentiated *Mx1*⁺ cells (Mx1+SMA-) or *αSMA⁺* cells (Mx1-SMA+) have much lower CCR5 expression (FIG. 4D). By contrast, a distinct *Mxl*⁺*αSMA*⁺ population (~0.01%) was not observed and there was significant upregulation of CCR5 in *Mx1*⁺ progenitors (26% of Mx1⁺140a⁺) in the BM (FIG. 4E). In addition, there was no differential expression of CXCR4 (receptor for CXCL12; a potential BM-SSC migratory factor) in *Mx1⁺αSMA⁺* P-SSCs, suggesting the presence of a CCR5-mediated regulatory mechanism in P-SSC migration (FIG. 4D, bottom).

It may be possible that *Mr1* does not label all SSCs in the BM and that *Mx1⁻* BM-SSCs can express CCR3/5. Using *LepR-Cre⁺Rosa26-Tomato⁺* reporter mice, generated to label postnatal BM-SSCs that are the major source of new osteoblasts and adipocytes in the BM (Zhou et al., 2014), *LepR⁺* cells were tested in the BM for expression of CCR5 and their contribution to osteolineage cells in injury repair. *In vivo* imaging and FACS analysis of these mice revealed that the *LepR⁺* cells are present in both the periosteum and the BM (FIG. 11A). When *LepR⁺* cells were tracked in the fracture injury, it was consistently observed that periosteal *LepR⁺* cells rapidly respond, proliferate, and contribute to injury healing, whereas no such migration and proliferation are observed in *LepR⁺* cells in the BM (FIG. 11B & FIG. 11C). In addition, the majority of *LepR⁺* periosteal cells express CCR5 (74%) with CD140a (82%), while *LepR⁺* cells in the BM have no such expression (FIG. 11D), further demonstrating that CCR5 expression may specify endogenous P-SSCs that are functionally distinct from BM-SSCs.

A previous study reported that *Prx1*-GFP⁺ cells in the periosteum have progenitor characteristics and contribute to injury repair (Ouyang et al., 2013). Therefore, to exclude any possible contamination of CCR5⁺ hematopoietic cells in the *Mx1*⁺*αSMA*⁺ P-SSC fraction, FACS analysis of periosteal cells from *Prx1*-CreER-GFP mice was performed and it was confirmed that *Prx1*-GFP⁺ P-SSCs (CD45⁻CD31⁻TER119⁻CD140a⁺GFP⁺) have high expression of CCR5 (86%) on the cell surface (FIG. 4F). Intravital imaging of Mx1/Tomato/Prx1CreER-GFP mice without tamoxifen induction revealed that nearly all *Prx1*-GFP⁺ periosteal cells overlap with *Mx1*⁺ P-SSCs (Tomato⁺) *in vivo* (FIG. 4G), supporting that *Mr1*⁺ P-SSCs with*Prx1*-GFP expression have specific CCR5 expression that may regulate early P-SSC migration toward injury sites *in vivo.*

### EXAMPLE 6

### CCL5 INDUCES THE IN VIVO MIGRATION OF P-SSCS

*In vitro* SSC migration followed by four-day colony formation assays with various bone growth factors revealed that tumor necrosis factor alpha (TNFα) and CCL5 (a common ligand for CCR3 and CCR5) (Blanpain et al., 2001) significantly enhance the migration capability of *Mr1*⁺ P-SSCs (FIG. 5A). To test if CCL5 induces the migration of P-SSCs toward fracture sites *in vivo, in vivo* imaging of *Mx1⁺αSMA*⁺ P-SSCs was performed one hour after the administration of CCL5 (2 µL, 10 ng/µL in Matrigel) at a fracture injury site. Visualization of *in vivo* migration of *Mx1⁺αSMA⁺* P-SSCs was possible by administering CCL5 locally (Figures 5B & FIG. 5C bottom), whereas in certain aspects various doses of TNFα or CXCL12 (10-50 ng in Matrigel) did not induce their migration *in vivo* (FIG. 5B & FIG. C top). CCL5 induces the directional migration of adjacent *Mx1*⁺ *αSMA⁺* P-SSCs toward the injury site while many distal P-SSCs remain stationary, with little movement. The total distance (*i.e.* multidimensional movement) of individual migrating cells (~20 cells/group) was assessed for 2 hours (1 minute/frame), and the average migration speed under CCL5 administration was 57 µm/h, while there was no distinct movement with TNFα and CXCL12 treatment in this particular experiment (FIG. 5B). CCL5 induces the directional migration of *Prx1*-GFP⁺ P-SSCs toward the injury site with a similar migration speed (FIG. 5C), supporting that these CCL5-mediated migrating cells are the non-hematopoietic *Mx1*⁺*αSMA*⁺ P-SSC fraction. Despite a wide variation in the migration of each *Mr1⁺αSMA⁺* P-SSCs due to a potential difference in CCL5 concentration at different locations at the time of imaging, the data indicate that P-SSCs are indeed dynamic and migrate upon CCL5 activation.

### EXAMPLE 7

### CCL5 IS USEFUL FOR BONE HEALING, AND LOCAL PROVISION OF CCL5 ACCELERATES HEALING OF AGED-BONE DEFECTS WITH INCREASED P-SSC RECRUITMENT.

The development of novel therapeutic avenues to accelerate early bone healing will eventually benefit patients with devastating skeletal injuries. Given the CCL5-mediated migration of *Mr1⁺αSMA⁺* P-SSCs and their recruitment toward injury sites, the *in vivo* contribution of CCL5 to the repair of fractured bones was assessed in an animal model. To test the physiologic importance of CCL5 in P-SSC migration and in injury repair, the bone healing of Ccl5-deficient mice was compared to that of WT litter mates. While there were no noticeable differences in bone parameters (BV/TV, travecular numbers and trabecular thickness) of 3-month-old Ccl5-deficient mice (data not shown), external callus mineralization (External BV/TV) and new bone formation (BV/TV) at defect sites 10 days after injury were significantly reduced (~35%, p < 0.01) in Ccl5-deficient mice compared to WT controls (FIG. 6A). Next, to test whether immune cells are the source of CCL5 for P-SSC migration and bone healing, WT mice were irradiated (9.5 Gy) and transplanted with Ccl5-deficient BM(10⁶ cells/mouse, Ccl5^{-/-}BMT) or WT BM as a control (WT-BMT). Five weeks after transplantation, a tibial drill-hole defect was induced and assessed bone healing 10 days after injury. A partial but significant delay in bone healing was observed in Ccl5^{-/-} BMT mice compared to WT-BMT mice (FIG. 6B). Consistent with previous reports showing CCL5 expression in multiple cell types upon injury (Aldinucci and Colombatti, 2014; Kovacic et al., 2010), these results imply that immune cells and other tissue cells express CCL5 at the injury site and contribute to P-SSC migration and bone healing.

Bone injuries were introduced to mouse calvaria of Mx1/Tomato/αSMA-GFP mice. Local treatment with CCL5 (+CCL5) or CXCL12 (+CXCL12) mixed with Matrigel (2 µL at 10 ng/µL) or Matrigel alone (CON) was given at the site of mechanical injury on days 0, 2, and 4. Treatment with CCL5 induced rapid recruitment of *Mx1*⁺*αSMA*⁺ P-SSCs at day 5 and substantially increased their number at day 10 with accelerated bone mineralization (FIG. 6C & FIG. 6D). Similarly, tibial injuries treated with CCL5 significantly increased early bone mineralization (FIG. 6E, D7), whereas treatment with a pharmacological CCR5 inhibitor (maraviroc) delayed bone healing (FIG. 6F). To confirm the findings, single-cell driven colonies from human periosteal tissues were cultured for 7 days. Cells were then analyzed for the expression of human SSC markers and CCR5. It was found that periosteal cells from both pooled culture and single-cell colonies express CCR5 and SSC markers (FIG. 6G), and that CCL5 treatment significantly induces human periosteal cell migration *in vitro* (FIG. 6H). These data indicate that local treatment with CCL5 enhances the recruitment of both mouse and human P-SSCs and may contribute to the recovery of fractured bones.

### EXAMPLE 8

### THE DELETION AND INHIBITION OF CCR5 DELAY P-SSC MIGRATION AND BONE REPAIR IN VIVO

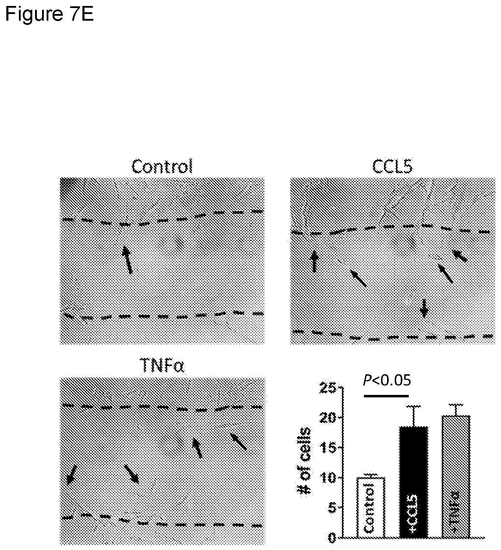

During live imaging of Mx1/Tom/SMA reporter mice treated with a pharmacological CCR5 inhibitor (FIG. 6F) a significant increase was noticed in the number and severity of periosteal resorption areas, whereas treatment with CCL5 lead to significantly greater recruitment of periosteal skeletal stem cells (PSSCs) and bone healing. To evaluate the function of CCR5 in bone healing, a tibial drill-hole defect (~1 mm) model was used to assess bone healing at 10 days post injury in Ccr5-deficient mice. Ccr5-deficient mice displayed a significant reduction in external callus formation (TV) and bone healing (BV/TV) of defect sites compared to WT littermate controls (FIG. 7A). To further evaluate the specific function of CCR5 in Mx1⁺αSMA⁺ P-SSCs migration during early bone healing, calvarial injuries were induced in Mx1/Tomato/αSMA-GFP mice (2 months after pIpC and WT-BMT) and treated with a pharmacological CCR5 inhibitor (Maravoric) every other day for 10 days. Inhibition of CCR5 substantially reduced the recruitment of Mx1⁺αSMA⁺ P-SSCs 5 and 10 days post injury (FIG. 7B). Furthermore, a significant reduction of bone healing (BV/TV) was observed at 7 days after injury (FIG. 7C). These data indicate that CCL5-CCR5 signaling is an important component of P-SSC migration in response to injury. To confirm the translational implications, primary cells isolated from collagenase-digested human periosteal tissue were analyzed for the expression of human SSC markers and CCR5. It was found that CD45⁻CD31⁻CD235a⁻CD140a⁺ human periosteal cells expressed CCR5 as well as previously defined human SSC markers such as CD105, CD146, and CD271 (FIG. 7D). Consistent with FIG. 6C, treatment with CCL5, but not with CXCL12, significantly induced human periosteal cell migration *in vitro* (FIG. 7E) and in a transwell migration assay (FIG. 7F). These data indicate that local treatment with CCL5 enhances the recruitment of both mouse and human P-SSCs and may contribute to the recovery of fractured bones.

### EXAMPLE 9

### IN VIVO IMAGING OF OSTEOCLASTIC RESPONSE TO CCL5 OR INHIBITION OF CCR5

The assessment of the *in vivo* contribution of CCL5 or CCR5 inhibitor (maraviroc) treatments on osteoclast function in relation to bone healing was performed. An animal model was developed in which it is possible to track the osteoclast response to bone injury. CathepsinK (Ctsk) is a known marker of post-natal osteoclasts. Developmentally both mesenchymal and hematopoietic cell linages are Tom+ in CtskCre/Tom reporter mice, which is evident by the imbedding of Tom+ cells within the bone and abundance of Tom+ cells within the bone marrow. To achieve selective labeling of hematopoietic linage osteoclast, a bone marrow transplant into wild type (WT) recipient mice is necessary. Seven-week-old WT mice were lethally irradiated with 9.50 Gy and the following day 10 million CtskCre/Tom bone marrow cells were transplanted (CtskCre-BM). The bone marrow cells were allowed to repopulate for at least 6 weeks prior to experimentation. To test if stimulation or inhibition of CCR5 prevents or induces osteoclast differentiation and/or migration in response to an injury, *in vivo* intravital imaging of Ctsk+ cell response to a calvarial injury was performed.

An injury was induced with a 27-gauge needle and mice received one of 3 treatments at the injury sight: 1) control (2 uL Matrigel) Day 0, 2, and 4; 2) CCL5 (2 uL, 10ng/uL in Matrigel) Day 0, 2, and 4; or 3) a CCR5 inhibitor (10 uL, 4.9 mM Maraviroc in Matrigel) on Day 0, 2, 4, 7, and 10 post-injury (FIG. 16A). *In vivo* imaging occurred on the day of injury as well as 4, 7, and 14 days post injury. Under control conditions no Ctsk⁺ cells were present at the injury sight the day of injury. By Day 4, Ctsk⁺ cells were present, and a 10-fold increase in cell number occurring 7 days post injury (p<0.01). Fourteen days post-injury the number of Ctsk⁺ cells were comparable to day 4 post injury. Treatment of the injury sight with CCL5 significantly reduced the number of Ctsk cells present at the injury on Day 7. Interestingly, ~50% more Ctsk cells were present day 14 with CCL5 treatment compared to the control (p<0.05), suggesting CCL5 lessens osteoclast response to injury. Treatment with the CCR5 inhibitor increased the number of Ctsk cells 7 days post injury. In addition to the number of Ctsk⁺ cells, osteoclastic resorption within the injury sight was apparent 4 days after injury and continued to expand with CCR5 inhibitor treatment compared to the control. Substantial resorption was not observed with CCL5 treatment until 14 days post injury, whereas resorption was present 7 days after injury in the control (FIG. 16B & FIG. 16C). Together these data support the hypothesis that CCL5 (or activation of CCR5) prevents osteoclast migration/differentiation in response to injury and inhibition of CCR5 stimulates osteoclast migration/differentiation and function.

### EXAMPLE 10

### INJURY REPAIR IN CCL5 ^{-/-} MICE

Based off findings that CCL5 treatment to bone injuries significantly increased the number of P-SSCs recruited to the injury sites and accelerates bone healing, the inventors next sought to determine if loss of CCL5 has deleterious effects on bone injury repair (see FIG. 12). Using age and sex-matched WT or 2-4-month-old CCL5^{-/-} mice, proximal tibia injuries were made on the anterior side of the proximal tibia using a 1.1 mm microdrill. Injured tibiae were collected ten-days post-injury and uCT imaging was performed. Injury repair was determined by the bone volume (BV) present in the total volume (TV) of the injury sites, and relative bone formation (BV/TV) was used to compare across groups to account for potential variability of the injury size. Loss of CCL5 resulted in an ~35% reduction in early bone mineralization (WT: 48.5 ± 3.5 *vs.* CCL5^{-/-}: 30.5 ± 2.6, p<0.01). These data indicate that loss of CCL5 negatively impacts early bone healing and therefore plays an integral part in bone healing.

## Claims

1. A composition comprising CCL5 for use in method of treating bone break, bone fracture, bone degeneration or osteoporosis in an individual, the method comprising administering the composition to the individual at a site in need thereof.

2. The composition for use according to claim 1, wherein the composition further comprises TNFα.

3. The composition for use according to claim 1 or 2, wherein the composition comprises a gel and/or scaffold.

4. The composition for use according to claim 3, wherein the gel comprises matrigel, hydrogel, periosteum, hydroxyapatite, tricalcium phosphate, polystyrene, poly-l-lactic acid, polyglycolic acid, poly-dl-lactic-co-glycolic acid, collagen, proteoglycans, alginate-based substrates, chitosan, collagen-GAG, extracellular matrix, or a combination thereof.

5. The composition for use according to any one of claims 1-4, wherein the administering comprises delivery of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 ng of CCL5 to the site in need.

6. The composition for use according to any one of claims 1-5, wherein the administering comprises at least one delivery to the individual.

7. The composition for use according to any one of claims 1-6, wherein the delivery is by injection.

8. The composition for use according to any one of claims 1-7, wherein the method comprises administration of CCL5 and at least one additional therapy.

9. The composition for use according to claim 8, wherein CCL5 and the at least one additional therapy are administered to the individual at the same time or at different times.

10. The composition for use according to claim 8 or 9, wherein the at least one additional therapy is administered to the individual in the composition comprising CCL5, or in a different composition from the composition comprising CCL5.

11. The composition for use according to any one of claims 8-10, wherein the additional therapy comprises surgical repair, fixation of a device to set the site in need, placing the individual in a cast or sling, or a combination thereof.

12. The composition for use according to any one of claims 1-11, wherein the site in need is monitored for bone regeneration.

13. The composition for use according to any one of claims 1-12, wherein the administration to the individual is prophylactic.

## Patentansprüche

1. CCL5 enthaltende Zusammensetzung für die Verwendung in einem Verfahren zum Behandeln von Knochenbruch, Knochenfraktur, Knochendegeneration oder Osteoporose bei einem Individuum, wobei das Verfahren das Verabreichen der Zusammensetzung an das Individuum an einer Stelle, die dies benötigt, umfasst.

2. Zusammensetzung für die Verwendung nach Anspruch 1, wobei die Zusammensetzung ferner TNFα umfasst.

3. Zusammensetzung für die Verwendung nach Anspruch 1 oder 2, wobei die Zusammensetzung ein Gel und/oder Gerüst umfasst.

4. Zusammensetzung für die Verwendung nach Anspruch 3, wobei das Gel Matrigel, Hydrogel, Periost, Hydroxyapatit, Tricalciumphosphat, Polystyrol, Poly-l-Milchsäure, Polyglykolsäure, Poly-dl-Milch-Co-Glykolsäure, Kollagen, Proteoglykane, Substrate auf Alginatbasis, Chitosan, Kollagen-GAG, extrazelluläre Matrix oder eine Kombination davon umfasst.

5. Zusammensetzung für die Verwendung nach einem der Ansprüche 1-4, wobei das Verabreichen die Abgabe von wenigstens etwa 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 ,40, 41, 42, 43, 44, 45, 46, 47, 48, 49, 50, 51, 52, 53, 54, 55, 56, 57, 58, 59, 60,61, 62,63, 64, 65, 66, 67, 68, 69, 70, 71,72, 73, 74, 75, 76, 77, 78, 79, 80, 81, 82, 83, 84, 85, 86, 87, 88, 89, 90, 91, 92, 93, 94, 95, 96, 97, 98, 99 oder 100 ng CCL5 an die Stelle mit Bedarf umfasst.

6. Zusammensetzung für die Verwendung nach einem der Ansprüche 1-5, wobei das Verabreichen wenigstens eine Abgabe an das Individuum umfasst.

7. Zusammensetzung für die Verwendung nach einem der Ansprüche 1-6, wobei die Abgabe durch Injektion erfolgt.

8. Zusammensetzung für die Verwendung nach einem der Ansprüche 1-7, wobei das Verfahren die Verabreichung von CCL5 und wenigstens einer zusätzlichen Therapie umfasst.

9. Zusammensetzung für die Verwendung nach Anspruch 8, wobei CCL5 und die wenigstens eine zusätzliche Therapie dem Individuum zu derselben Zeit oder zu unterschiedlichen Zeiten verabreicht werden.

10. Zusammensetzung für die Verwendung nach Anspruch 8 oder 9, wobei die wenigstens eine zusätzliche Therapie in der Zusammensetzung, die CCL5 umfasst, oder in einer Zusammensetzung an das Individuum verabreicht wird, die sich von der Zusammensetzung, die CCL5 umfasst, unterscheidet.

11. Zusammensetzung für die Verwendung nach einem der Ansprüche 8-10, wobei die zusätzliche Therapie eine chirurgische Reparatur, eine Fixierung einer Vorrichtung, um die Stelle mit Bedarf zu richten, das Platzieren des Individuums in einen Gips oder eine Schlinge oder eine Kombination davon umfasst.

12. Zusammensetzung nach einem der Ansprüche 1-11, wobei die Stelle mit Bedarf auf Knochenregeneration überwacht wird.

13. Zusammensetzung nach einem der Ansprüche 1-12, wobei die Verabreichung an das Individuum prophylaktisch ist.

## Revendications

1. Composition comprenant une cytokine CCL5 destinée à une utilisation dans une méthode de traitement d'os cassé, de fracture osseuse, de dégénérescence osseuse ou d'ostéoporose chez un individu, la méthode comprenant l'administration de la composition à l'individu au site en ayant besoin.

2. Composition destinée à une utilisation selon la revendication 1, ladite composition comprenant en outre une cytokine TNFα.

3. Composition destinée à une utilisation selon la revendication 1 ou 2, ladite composition comprenant un gel et/ou un échafaudage.

4. Composition destinée à une utilisation selon la revendication 3, dans laquelle le gel comprend : matrigel, hydrogel, périoste, hydroxyapatite, phosphate tricalcique, polystyrène, acide poly-l-lactique, acide polyglycolique, acide poly-dl-lactique-co-glycolique, collagène, protéoglycanes, substrats à base d'alginate, chitosane, collagène-GAG, matrice extracellulaire ou une combinaison de ceux-ci.

5. Composition destinée à une utilisation selon l'une quelconque des revendications 1 à 4, ladite administration comprenant l'administration d'au moins environ 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 ng de CCL5 au site en ayant besoin.

6. Composition destinée à une utilisation selon l'une quelconque des revendications 1 à 5, ladite administration comprenant au moins une administration à l'individu.

7. Composition destinée à une utilisation selon l'une quelconque des revendications 1 à 6, ladite administration se faisant par injection.

8. Composition destinée à une utilisation selon l'une quelconque des revendications 1 à 7, ladite méthode comprenant l'administration de CCL5 et d'au moins un traitement supplémentaire.

9. Composition destinée à une utilisation selon la revendication 8, ladite CCL5 et l'au moins un traitement supplémentaire étant administrés à l'individu au même moment ou à des moments différents.

10. Composition destinée à une utilisation selon la revendication 8 ou 9, ledit au moins un traitement supplémentaire étant administré à l'individu dans la composition comprenant de la CCL5 ou une composition différente de la composition comprenant de la CCL5.

11. Composition destinée à une utilisation selon l'une quelconque des revendications 8 à 10, ledit traitement supplémentaire comprenant une intervention chirurgicale, la fixation d'un dispositif pour réduire le site en ayant besoin, la pose d'un plâtre ou d'une écharpe chez l'individu, ou une combinaison de ceux-ci.

12. Composition destinée à une utilisation selon l'une quelconque des revendications 1 à 11, mettant en œuvre une surveillance de la régénération osseuse audit site en ayant besoin.

13. Composition destinée à une utilisation selon l'une quelconque des revendications 1 à 12, ladite administration à l'individu étant prophylactique.
